# EUROPEAN PATENT SPECIFICATION

(11) **EP 4 196 195 B1**
(45) Date of publication and mention of the grant of the patent: **25.02.2026**
(21) Application number: 21762644.9
(22) Date of filing: 10.08.2021
(51) Int. Cl.: A61M 5/315, A61M 5/50

(54) **INJECTION DEVICE AND DOSE LIMITING MECHANISM**
INJEKTIONSVORRICHTUNG UND DOSISBEGRENZUNGSMECHANISMUS
DISPOSITIF D'INJECTION ET MÉCANISME DE LIMITATION DE DOSE

(30) Priority: 12.08.2020 EP 20315383
(43) Date of publication of application: 21.06.2023
(73) Proprietor: Sanofi, 75017 Paris (FR)
(72) Inventor: JUGL, Michael, 65926 Frankfurt am Main (DE); BLANCKE, Stefan, 65926 Frankfurt am Main (DE)
(74) Representative: Weilnau, Carsten
(86) International application number: PCT/EP2021/072229
(87) International publication number: WO 2022/034052

(56) References cited:
- US-A1- 2011 245 780
- US-A1- 2012 010 575
- US-A1- 2015 290 395

## Description

### Field

The present disclosure is generally directed to an injection device, e.g. a drug delivery device for automatic spring driven injection of a liquid drug, i.e. a medicament, by which doses of an individual size can be set by a user. The disclosure further relates to a dose limiting mechanism for such an injection device.

### Background

Drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament. The present disclosure is hence not directed to so called fixed dose devices which only allow dispensing of a predefined dose without the possibility to increase or decrease the set dose.

There are basically two types of drug delivery devices: resettable devices (i.e., reusable) and non-resettable (i.e., disposable). For example, disposable drug delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is in general applicable for both types of devices, i.e. for disposable devices as well as for reusable devices.

A further differentiation of drug delivery device types refers to the drive mechanism: There are devices which are manually driven, e.g. by a user applying a force to an injection button, devices which are driven by a spring or the like and devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting. Further types of energy storage may comprise compressed fluids or electrically driven devices with a battery or the like.

Although many aspects of the present disclosure are applicable for all of these types of devices, i.e. for devices with or without a drive spring or the like energy storage. These types of delivery devices generally comprise three primary elements: a cartridge section that includes a cartridge often contained within a housing or body or holder; a needle assembly connected to one end of the cartridge section; and a dosing section connected to the other end of the cartridge section. A cartridge (often referred to as an ampoule) typically includes a reservoir that is filled with a medication (e.g., insulin), a movable rubber type bung or stopper located at one end of the cartridge reservoir, and a top having a pierceable rubber seal located at the other, often necked-down, end. A crimped annular metal band is typically used to hold the rubber seal in place. While the cartridge housing may be typically made of plastic, cartridge reservoirs have historically been made of glass.

The needle assembly is typically a replaceable double-ended needle assembly. Before an injection, a replaceable double-ended needle assembly is attached to one end of the cartridge assembly, a dose is set, and then the set dose is administered. Such removable needle assemblies may be threaded onto, or pushed (i.e., snapped) onto the pierceable seal end of the cartridge assembly.

The dosing section or dose setting mechanism is typically the portion of the device that is used to set (select) a dose. During an injection, a lead screw, a plunger or piston rod contained within the dose setting mechanism presses against the bung or stopper or piston of the cartridge. This force causes the medication contained within the cartridge to be injected through an attached needle assembly. After an injection, as generally recommended by most drug delivery device and/or needle assembly manufacturers and suppliers, the needle assembly is removed and discarded.

The dosing section of drug delivery devices for selecting and dispensing a number of user variable doses of a medicament often comprises a display for indicating the selected dose to a user. This is especially important where a user may select a different dose each time depending on the state of health. There are mechanical displays, e.g. a drum with printed numbers on its outer surface, wherein the number corresponding to the actually selected dose is visible through a window or opening in the device. Although such mechanical displays are simple and reliable, they usually require a relatively large construction space which makes the devices bulky.

Drug delivery devices or injection devices for setting of a dose of variable size and for subsequently injecting or dispensing of such a dose may be also equipped with a so-called last dose limiting mechanism. A last dose limiting mechanism typically keeps track of the total amount of a medicament dispensed the from a medicament container or cartridge. The last dose limiting mechanism typically serves to prevent setting of a dose that would exceed the residual amount of the medicament provided in the medicament container or cartridge.

Some medicaments and hence some medication schedules require a regular injection of the liquid medicament. Since the amount of medicament provided in the medicament container or cartridge is generally sufficient to conduct multiple injections over a comparatively long time interval it should be ensured, that a medicament cannot be injected any longer ones an expiry date of the medicament has lapsed.

With some medicaments the medicament container and hence the medicament located therein should be used-up within a predefined time interval after a first use of the medicament. For instance, the lifecycle of a cartridge should not exceed a predefined time interval, e.g. several days or weeks.

Furthermore, and as a dose of the medicament should be dispensed or injected regularly, e.g. ones or twice a day it should be ensured that a user regularly using the injection device cannot artificially or unsuitably prolong the lifecycle of the medicament container or cartridge.

It is therefore desirable to provide an injection device with a dose limiting mechanism operable to limit a total number of doses dispensable by the injection device. It is a further aim to provide a dose limiting mechanism operable to limit a total number of doses dispensable by an injection device and being suitable for a large variety of injection devices. The injection device should provide setting and dispensing of doses of variable, and hence user-settable size.

The dose limiting mechanism and the injection device should provide a functionality to prevent setting and/or dispensing of a dose of the medicament after a predefined number of dose dispensing procedures have been conducted. The dose limiting mechanism and the injection device should provide a limitation of the total number of doses dispensable or injectable by the injection device irrespective of the total amount of medicament dispensed or injected by consecutive dose dispensing procedures. Dose limiting mechanisms known from the prior art are published in US2011/245780A1, US2015/290395A1 and US2012/010575A1.

### Summary

In one aspect there is provided a dose limiting mechanism for an injection device, such as a pen-type injector. The dose limiting mechanism is configured for arrangement inside a housing of the injection device. The dose limiting mechanism is operable to limit a total number of doses dispensable or injectable by the injection device. The dose limiting mechanism comprises a first elongated component extending in a longitudinal direction and comprising a first profiled structure. The dose limiting mechanism further comprises a second elongated component coaxial or parallel to the first elongated component. The second elongated component comprises a second profiled structure facing towards the first profiled structure.

The dose limiting mechanism further comprises a dose limiter arranged between the first elongated component and the second elongated component. The dose limiter comprises a first profile section and a second profile section. The first profile section is engageable with the first profiled structure to transfer a longitudinal movement of the first elongated component to the dose limiter when the first elongated component is subject to a longitudinal movement in a distal direction relative to the second component during or for dispensing of a dose.

The second profile section of the dose limiter is engageable with the second profiled structure of the second elongated component to retain a longitudinal position of the dose limiter relative to the second component when the first component is subject to a longitudinal movement in a proximal direction relative to the second component.

In this way the dose limiter is operable to follow a longitudinally and distally directed displacement of the first elongated component relative to the second elongated component. In case that the first elongated component is subject to an oppositely directed longitudinal displacement, hence along the proximal direction the dose limiter is retained by the second longitudinal component.

Typically, and when implemented in an injection device the first elongated component is subject to a distally directed displacement relative to the second elongated component when a dose dispensing action is triggered, i.e. when a trigger of the injection device is depressed by a user. The first elongated component may be subject to an oppositely directed movement, hence along the proximal direction, when a trigger of the injection device is released, e.g. at the end of a dose dispensing or dose injecting procedure. Such a proximally directed motion may be induced or caused by a spring element.

In typical configurations the first elongated component is subject to a longitudinal forth and back movement relative to the second elongated component during or for dispensing of a dose of the medicament. With some examples the first elongated component is subject to a distally directed movement relative to the second elongated component at the beginning of a dose dispensing action and the first elongated component is subject to a proximally directed longitudinal displacement relative to the second elongated component when a dose dispensing action is interrupted or when a dose dispensing procedure has terminated.

The first profiled structure of the first elongated component is engageable with the first profile section of the dose limiter such that a distally directed longitudinal displacement of the first elongated component is equally or unalterably transferred to the dose limiter as the first elongated component is moved in distal direction relative to the second elongated component.

In the opposite sense of movement, hence when the first elongated component is subject to a proximally directed movement relative to the second elongated component the mutual engagement of the first profiled structure and the first profile section is such, that the first profiled structure is allowed to move proximally relative to the first profile section. In this way, the first elongated component is allowed to move in proximal direction relative to the dose limiter.

For this the dose limiter is operably engaged with the second profiled structure of the second elongated component through its second profile section. In other words, the mutual engagement of the second profiled structure of the second elongated component and the second profile section of the dose limiter is such that the dose limiter is always allowed to move in distal direction relative to the second elongated component but is hindered to move proximally relative to the second elongated component.

The mutual engagement between the first profile section of the dose limiter and the first profiled structure of the first elongated component supports and enables a proximally directed movement of the first elongated component relative to the dose limiter but is operable to prevent a distally directed displacement of the first elongated component relative to the dose limiter. As the first elongated component is subject to a distally directed movement relative to the second elongated component the dose limiter has to follow this distally directed movement.

Typically, the first elongated component is movable from a first longitudinal position, e.g. an initial position, to a second longitudinal position, e.g. a trigger position, along the distal direction relative to the second elongated component and/or relative to the housing of the injection device. When arriving at the second longitudinal position the dose limiter, which has been also moved from a first longitudinal position towards and into a second longitudinal position, is longitudinally secured to the second elongated component at least with regards to a proximally directed displacement. As the first elongated component should become subject to a return movement from the second longitudinal position along the proximal direction towards and into the first longitudinal position the second profile section of the dose limiter is longitudinally engaged with the second profiled structure of the second elongated component such that the dose limiter is longitudinally retained at the second elongated component.

According to a further example the first profiled structure comprises a regularly shaped profiled structure extending along the longitudinal direction. The same applies for the second profiled structure. Also, the second profiled structure extends along the longitudinal direction and comprises a regular profile as seen in longitudinal direction.

According to a further example and each time the first elongated component is subject of a longitudinal distally directed movement from the first position towards and into the second position the dose limiter is moved accordingly in distal direction. Typically, the longitudinal distance between the first longitudinal position and the second longitudinal position of the first elongated component defines a step size. Each time the first elongated component is subject to a distally directed movement from the first longitudinal position towards and into the second longitudinal position the dose limiter is subject to a distally directed movement in by the step size.

During an oppositely directed return movement of the first elongated component relative to the second elongated component the dose limiter is retained in longitudinal direction with and/or by the second longitudinal component. With a subsequent and distally directed longitudinal displacement of the first elongated component relative to the second elongated component from the first longitudinal position towards and into the second longitudinal position the dose limiter is again subject to a further distally directed movement in accordance to the step size. Typically, and with each distally directed longitudinal movement of the first elongated component relative to the second elongated component the dose limiter is subject to a well-defined step-wise and discrete longitudinal displacement in distal direction.

With some examples and when for instance the first elongated component is longitudinally engaged with a trigger of the injection device and when the second elongated component is longitudinally retained to the housing of the injection device the dose limiter is subject to a stepwise distally directed movement relative to the second elongated component and hence relative to the housing each time the trigger of the injection device is depressed. In this way a longitudinal position of the dose limiter relative to the second elongated component or relative to the housing is directly indicative to the total number of dispensing actions that have been conducted by the injection device.

With some examples there is provided an end stop on one of the first elongated component, the second elongated component or on the housing of the injection device. The end stop is configured to engage with the dose limiter. When the dose limiter is in an engaging configuration with the end stop the dose limiter and/or the first elongated component is at least one of longitudinally retained or rotationally retained relative to the second elongated component and/or relative to the housing. With some examples and when the dose limiter is in an engagement configuration with the end stop a movability of the second elongated component, e.g. a rotation of the second elongated component relative to the first elongated component and/or relative to the housing will be blocked.

Accordingly, and when in the blocking configuration, in which the dose limiter is engaged with the end stop, setting of a dose and/or dispensing of a dose will be no longer possible with the injection device.

According to the claimed invention, the first profiled structure comprises a number of stop faces consecutively arranged in longitudinal direction and facing in the distal direction. The stop faces of the first profiled structure are configured to longitudinally engage or to longitudinally abut with the first profile section of the dose limiter. In this way and when a stop face of the first profiled structure is in longitudinal or axial abutment or engagement with the first profile section of the dose limiter a longitudinally and distally directed displacement of the first elongated component relative to the second elongated component is transferred to the dose limiter.

The first profiled structure comprises a number of regularly, e.g. equidistantly arranged stop faces along the longitudinal direction. Typically, the consecutive or adjacently located stop faces are separated by a longitudinal distance in accordance to the step size as defined by the longitudinal distance between the first longitudinal position, in which the first elongated component is located in an initial position and the second longitudinal position, in which the first elongated component is in the trigger position.

Typically, the number of stop faces correlates with or defines the total number of doses dispensable by the injection device. Before using the injection device for a first time a first stop face of the first profiled structure may be in longitudinal abutment or longitudinal engagement with the first profile section of the dose limiter.

During dose dispensing the dose limiter is shifted or moved in distal direction by the predefined step size as the first elongated component is subject to a distally directed movement from the first longitudinal position towards and into the second longitudinal position. When the first elongated component is subject to a proximally directed return movement a second stop face of the profiled structure may engage or align with the first profile section of the dose limiter at least when the first elongated component returns to and reaches the first longitudinal position or initial position, e.g. at the end of a dose injection procedure, hence, when a trigger of the injection device is released.

Now and with a subsequent dose dispensing procedure it will be this second stop face of the profiled structure that serves to drag or to move the dose limiter further in distal direction by the predefined step size. After a second dose dispensing action the first elongated component is again subject to a proximally directed return movement. At the end of this second return movement a third stop face of the first profiled structure longitudinally engages or aligns with the first profile section of the dose limiter.

Typically, the longitudinal distance between consecutively arranged stop faces of the first profiled structure correlates or substantially equals the step size as governed by the longitudinal distance between the first longitudinal position and the second longitudinal position of the first elongated component in the course of initiating and/or terminating a dose dispensing procedure.

According to a further example the first profile section comprises a first counter stop face facing in the proximal direction and being configured to engage with the stop face(s) of the first profile fracture. In this way any distally directed displacement of the first elongated component relative to the second elongated component can be unalterably transferred to the dose limiter. Prior to a first dose dispensing procedure the first counter stop face of the first profile section will be in axial abutment with the distally facing first stop face of the first profiled structure. After termination of a first dispensing procedure the first counter stop face of the first profile section will be in longitudinal or axial abutment, engagement or alignment with a second or consecutive stop face of the first profiled structure of the first elongated component and so on.

In this way and with each distally directed stepwise movement of the first elongated component relative to the second elongated component the first counter stop face of the first profile section of the dose limiter engages with a consecutive stop face of the number of stop faces of the first profiled structure.

According to another example the second profiled structure of the second elongated component comprises a number of retaining faces. The retaining faces are consecutively arranged in longitudinal direction and face in the distal direction. Typically, the retaining faces of the second profiled structure are arranged in a regular, e.g. equidistant manner along the longitudinal direction of the second elongated component. A longitudinal distance between consecutively or adjacently arranged distally facing retaining faces of the second profiled structure typically coincides or correlates with the step size as governed by the longitudinal distance between the first and second longitudinal positions of the first elongated component.

Typically, and prior to dispense a first dose of the medicament the second profiled structure is in longitudinal or axial abutment with a first retaining face. As the dose limiter is dragged or pushed in distal direction through a distally directed movement of the first elongated component relative to the second elongated component the second profile section of the dose limiter will engage with a second retaining face of the second profiled structure. The second retaining face begin distally offset from the first retaining face by a well-defined axial or longitudinal distance as governed by the step size. In this way and when the second profile section engages a second distally facing retaining face of the second profiled structure the dose limiter it is effectively hindered to move in proximal direction relative to the second profiled structure and hence relative to the second elongated component.

Consequently, and when the dose limiter has moved a well-defined step size in distal direction relative to the second elongated component its second profile section will engage with a retaining face of the second profiled structure and will be thus hindered to move in proximal direction relative to the second elongated component.

According to a further example the second profile section of the dose limiter comprises a second counter stop face facing in the proximal direction and being configured to engage with the retaining faces of the second profiled structure. When engaged with the distally facing retaining face of the second profiled structure the second counter stop face serves to block a proximally directed movement of the second profile section and hence of the entire dose limiter relative to the second elongated component.

According to further examples the mutual engagement between the first profiled structure and the first profile section provides a unidirectional ratchet allowing and enforcing that a distally directed movement of the first elongated component is transferred to the dose limiter. The mutual engagement further supports and allows for a proximally directed movement of the first elongated component relative to the dose limiter and hence relative to the second elongated component.

The mutual engagement of the second profile section of the dose limiter with the second profiled structure of the second elongated component provides a kind of a unidirectional ratchet that supports a distally directed movement of the dose limiter relative to the second elongated component but prevents and blocks a proximally directed movement of the dose limiter relative to the second elongated component.

In other words, the mutual engagement of the first profile section with the first profiled structure supports and allows a proximally directed displacement of the first elongated component relative to the dose limiter but prevents a distally directed displacement of the dose limiter relative to the first elongated component. A distally directed displacement of the first elongated component is unalterably transferrable to the dose limiter.

Typically, the first and second profiled structure as well as the complementary shaped first and second profile sections may comprise mutually corresponding pairs of protrusions and recesses or pocket holes. With some examples the first profiled structure may comprise a number of teeth, e.g. radially or transversally protruding from the first elongated component. Alternatively, the first profiled structure comprises holes provided with a number of stop faces, e.g. extending perpendicular, transversally or in radial direction relative to the elongation of the first elongated component.

The first profile section of the dose limiter is shaped complementary. It may either comprise a complementary-shaped tooth with a counter stop face protruding perpendicularly, transversally or in radial direction relative to the elongation of the first elongated component. Likewise, also the second profiled structure may comprise either a toothed structure with teeth protruding perpendicularly, transversally or in radial direction relative to the elongation of the second elongated component.

Alternatively, the second profiled structure comprises stop faces extending substantially perpendicularly or in radial direction relative to the elongation of the second elongated component. When the retaining faces of the second profiled structure are provided as sidewalls of recesses of the second profiled structure the correspondingly shaped second counter stop face of the second profile section protrudes radially, transversally or perpendicularly with regard to the elongation of the second elongated component.

Vice versa, when the second profiled structure comprises a radially protruding toothed structure, wherein the retaining faces are provided on radially protruding teeth it is conceivable that the dose limiter and hence the complementary shaped second profile section comprises the second counter stop face either in a recessed portion or on a protruding portion.

In either way and when the first profiled structure comprises a number of recesses the first profile section comprises a protrusion. Vice versa when the first profiled structure comprises a number of protruding teeth the first profile section may comprise a recess to engage with the teeth or it may alternatively comprise a protruding tooth to engage with the teeth of the first profiled structure. The same applies to the mutual engagement between the second profiled structure and the second profile section.

According to another example the first elongated component and the second elongated component being rotatable relative to each other during setting of the dose. The first elongated component and the second elongated component being further rotationally locked during dispensing of the dose.

There may be provided a clutch mechanism operating between the first elongated component and the second elongated component. The clutch can be activated or deactivated through an axial or longitudinal sliding movement of the first elongated component relative to the second elongated component. Typically, and when the first elongated component is in the first longitudinal position or in the initial position, in which a trigger of the injection device is not depressed the first and the second elongated component are free to rotate relative to each other. It is upon a distally directed movement of the first elongated component relative to the second elongated component, e.g. induced by depressing of the trigger of the injection device, that the first elongated component is rotationally locked to the second elongated component.

With some examples the first elongated component comprises a tubular sleeve and the second elongated component also comprises a tubular sleeve. The first and the second elongated components may be arranged co-axially. They may be arranged in a nested manner, wherein one of the first elongated component and the second elongated component is located radially inside the other one of the first elongated component and the second elongated component. With such implementations the dose limiter is typically arranged radially between the first and the second elongated component. With some examples the dose limiter comprises a closed ring structure. With other examples the dose limiter comprises an open ring structure or a semicircular structure.

According to another example the dose limiter is rotationally locked to one of the first elongated component and the second elongated component. The dose limiter is further rotatable to the other one of the first elongated component and the second elongated component. Typically, the dose limiter may be rotationally locked to one of the first and second elongated components by way of a spline feature. Accordingly, one of the dose limiter and the respective elongated component comprises a radial protrusion slidably engaged with a longitudinally extending recess or groove of the other one of the dose limiter and the respective elongated component. In this way a rotational interlock between the dose limiter and one of the first and second elongated components can be provided.

With some examples the dose limiter is permanently rotationally locked to the second elongated component. It may slide along the second elongated component in longitudinal direction.

According to a further example at least one of the first elongated component and the second elongated component comprises an end stop configured to engage with the dose limiter. The end stop may be provided at or near a distal end of the first elongated component and the second elongated component. When implemented in an injection device the end stop may be alternatively provided on or by the housing of the injection device or by some other component non-movably fixed to the housing of the injection device.

Typically and when the end stop is provided on the first elongated component it may serve to prevent or to disable a proximally directed movement of the first elongated component relative to the dose limiter and hence relative to the second elongated component, provided that the dose limiter is in proximal axial engagement with the second elongated component. With such a configuration a trigger or trigger button longitudinally connected to the first elongated component and after having being depressed in distal direction may be hindered to return into an initial position e.g. at the end of a dose dispensing procedure. This will then be a clear indication to the user, that an allowable or intended total number of dose injection procedures has been conducted.

With another example and when the end stop is provided on or by the second elongated component it may be operable to block a further distally directed movement of the dose limiter beyond the end stop induced by the first elongated component. Hence, when the dose limiter has reached the end stop on or at the second elongated component it is hindered to move any further in distal direction relative to the second elongated component. In this way and since the dose limiter is blocked from moving in distal direction relative to the second elongated component a distally directed movement of the first elongated component relative to the second elongated component is effectively blocked.

In this way, the first elongated component is hindered to move distally relative to the second elongated component. When implemented in the injection device a trigger of the injection device is locked against a distally directed depression induced by a user.

Accordingly, and with a further embodiment the end stop of the first elongated component is configured to prevent a proximally directed longitudinal movement of the first elongated component relative to the dose limiter or relative to the second elongated component when engaged with the dose limiter. Here, the end stop may comprise a proximally facing abutment face to engage with a distally facing abutment face of the dose limiter. The mutual abutment or engagement of mutually corresponding abutment faces of the first elongated component and the dose limiter prevents and blocks a proximally directed movement of the first elongated component relative to the dose limiter.

According to a further example the end stop of the second elongated component is configured to prevent or to block a distally directed longitudinal movement of the first elongated component relative to the dose limiter or relative to the second elongated component when engaged with the dose limiter.

Here, the dose limiter may comprise a distally facing abutment face configured to get in longitudinal abutment or longitudinal engagement with a proximally facing abutment face of the end stop of the second elongated component. In this way the mutual abutment of the correspondingly shaped abutment faces prevents and blocks a distally directed displacement of the dose limiter beyond the end stop of the second elongated component.

Accordingly, and when the first elongated component, in particular the first profiled structure thereof is in longitudinal abutment or engagement with the first profile section of the dose limiter the first elongated component cannot be moved any further in distal direction relative to the dose limiter and hence relative to the second elongated component. Any further or repeated dose dispensing action is therefore blocked.

According to a further example the end stop and the dose limiter are configured to rotationally interlock when the dose limiter overlaps in longitudinal direction with the end stop. In this way and when reaching a stop configuration the dose limiter is hindered from rotating relative to the end stop. This is of particular benefit when the dose limiter is permanently rotationally engaged or rotationally locked e.g. to the second elongated component but is free to rotate relative to the first elongated component. Here, the end stop will be provided on a predefined longitudinal position of the first elongated component. When the dose limiter reaches the respective end stop configuration it rotationally locks to the first elongated component.

Since the dose limiter is rotationally interlocked or rotationally constrained to the second elongated component also the second elongated component will rotationally interlock to the first elongated component. With some examples, wherein the first elongated component is hindered from rotating during a dose setting procedure while the second elongated component has to be rotated for setting of a dose a further dose setting action can be blocked and impeded as soon as the dose limiter rotationally engages with the end stop.

With another example the dose limiter is permanently rotationally locked to the first elongated component and is free to rotate relative to the second elongated component. Here, the end stop configured to rotationally engage with the dose limiter is provided on the second elongated component. In this way a likewise rotational interlock between the first and second elongated components will be obtained when the dose limiter rotationally interlocks with the end stop.

According to another example one of the end stop and the dose limiter comprises at least one locking tooth and the other one of the end stop and the dose limiter comprises a gear ring to rotationally engage with the at least one locking tooth.

That component provided with the at least one locking tooth may also comprise at least a second locking tooth located at a circumferential offset from a first locking tooth also configured to rotationally engage with the gear ring. In this way, at least a twofold rotational interlock between the end stop and the dose limiter can be obtained. This provides a rather robust and failure safe mutual interlocking configuration between the dose limiter and the end stop.

When the dose limiter and the end stop are configured to rotationally interlock the first and second elongated components are typically provided as a tubular shaped sleeve, respectively. With some examples the at least one locking tooth or a number of locking teeth is or are provided on an outer circumference of the first elongated component thereby providing or constituting the end stop. Here, the gear ring is provided on an inside surface of an annular or semicircular shaped dose limiter.

With other examples a gear ring may be provided on an outside surface of the first elongated component whereas the at least one or several locking tooth or locking teeth are provided on an inside surface of the dose limiter.

With another example the first elongated component comprises a drive sleeve movable from a first longitudinal position to a second longitudinal position along the distal direction relative to the housing of the injection device, wherein when in the first longitudinal position the drive sleeve being rotationally constrained to the housing and wherein when in the second longitudinal position the drive sleeve being free to rotate relative to the housing.

The dose limiting mechanism or drive mechanism of the injection device may further comprise a lead screw configured to operably engage with a piston of the cartridge in order to displace the piston relative to a barrel of the cartridge for the purpose of expelling of the dose of the medicament from the cartridge.

The drive sleeve may be operably engaged with the lead screw such that a rotation of the drive sleeve relative to the housing of the injection device leads to or causes a distally directed movement of the lead screw relative to the housing and/or relative to the barrel of the cartridge.

According to another example the second elongated component comprises a number sleeve or a dose scale drum longitudinal constrained to the housing and being free to rotate relative to the housing. The number sleeve typically comprises a consecutive sequence of numbers being indicative of a size of a dose currently set or dispensed. Typically, only a portion of the number sleeve is visible from outside the housing of the injection device. For setting of a dose, the number sleeve is subject to a rotation relative to the housing along a first direction. During or for dispensing of the dose the number sleeve is subject to a rotation in the opposite direction, hence along a second direction. During setting of a dose, the first elongated component, e.g. in form of the drive sleeve is rotationally locked to the housing while the second elongated component, e.g. in form of the number sleeve, is subject to a rotation relative to the housing and relative to the first elongated component.

At the beginning of a dose dispensing action and wherein a trigger of the injection device is depressed by a user, the first elongated component is subject to a longitudinally and distally directed movement relative to the housing and/or relative to the second elongated component thereby pushing or dragging the dose limiter one step further in distal direction until the second longitudinal position has been reached, in which the dose limiter is engaged with the second elongated component and in which the dose limiter is hindered to move in proximal direction relative to the second elongated component.

During or for the purpose of dispensing of the dose a clutch between the first elongated component and the second elongated component may be closed so that the first and second elongated component, i.e. the number sleeve and the drive sleeve rotate in unison.

The distally directed movement of the first elongated component and hence of the drive sleeve may also open a clutch by way of which the first elongated component is rotationally constrained or rotationally locked to the housing.

According to another example a longitudinal movement of the first elongated component relative to the second elongated component or a longitudinal movement of the first elongated component relative to the housing of the injection device is controlled by a dispensing spring. Hence, the first elongated component may be longitudinally engaged with the dispensing spring operating as a return spring. The first elongated component may be movable from the first longitudinal position towards and into the second longitudinal position through a biasing of the dispensing or return spring.

When released in the second longitudinal position the dispensing or return spring is operable to move the first elongated component back into the first longitudinal position.

In another aspect the present disclosure relates to an injection device for setting and dispensing of a dose of a medicament. The injection device comprises a housing to accommodate a cartridge filled with the liquid medicament. The injection device further comprises a lead screw to operably engage with the cartridge for expelling of a dose of the medicament from the cartridge. The injection device further comprises a dose dial to set or to dial a dose of variable size. Typically, the dose dial is rotationally supported on the housing of the injection device. It may be provided at or near a proximal end of the housing.

The injection device further comprises a trigger to initiate dispensing of the dose. The trigger may be also implemented and used to control dispensing of the dose. Furthermore, the injection device comprises a dose limiting mechanism as described above. Here, the first elongated component of the dose limiting mechanism is longitudinally engaged or longitudinally connected with the trigger of the injection device. The second elongated component of the dose limiting mechanism is longitudinally constrained to the housing. In this way and when the trigger of the injection device is depressed, typically in distal direction, the first elongated component is subject to a respective stepwise movement in distal direction relative to the second elongated component. In this way, the dose limiter is moved by a well-defined step in distal direction until it engages with the second elongated component by way of which the dose limiter is hindered to move proximally as the trigger is released, e.g. at the end of a dose dispensing procedure. In this way the first elongated component is allowed to return into the first longitudinal position towards the proximal direction.

According to a further example the injection device comprises the above-mentioned cartridge filled with the liquid medicament and being arranged inside the housing. The cartridge may be readily assembled inside the injection device. The injection device may be implemented as a pen-type injector. It may be implemented as a disposable device, when the cartridge with the medicament is assembled inside the housing of the injection device.

With other examples the injection device is implemented as a reusable device. Here, the housing of the injection device can be opened or can be disassembled to enable a replacement of the cartridge.

The injection device may be implemented as an automated spring driven injection device. Here, the injection device may comprise a drive spring, e.g. implemented as a torsion spring. The drive spring may be configured to store mechanical energy. The driving spring may be biased during setting of a dose, e.g. when the dose dial of the injection device is rotated in a first direction, e.g. in a dose incrementing direction. The drive spring, typically pre-biased will be biased further by the dose dialing action. Upon depression of the trigger mechanical energy stored in the drive spring and e.g. harvested during setting of the dose will be released. The energy released from the drive spring may be sufficient to rotate the drive sleeve and to move the lead screw in distal direction or dispensing of the dose of the medicament

In the present context the term 'distal' or 'distal end' relates to an end of the injection device that faces towards an injection site of a person or of an animal. The term 'proximal' or 'proximal end' relates to an opposite end of the injection device, which is furthest away from an injection site of a person or of an animal.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

It will be further apparent to those skilled in the art that various modifications and variations can be made to the present disclosure without departing from the scope of the disclosure. Further, it is to be noted, that any reference numerals used in the appended claims are not to be construed as limiting the scope of the disclosure. The invention is defined by the appended claims.

### Brief description of the drawings

In the following, numerous examples of the dose limiting mechanism and of an injection device will be described in greater detail by making reference to the drawings, in which:
Fig. 1 shows an exploded view of an example of an injection device,
Fig. 2 shows an exploded view of another example of an injection device,
Fig. 3 a perspective view of the sliding element of the device in Fig. 2,
Fig. 4 a perspective view of the number sleeve of the device in Fig. 2,
Fig. 5 a perspective view of another section of the number sleeve in Fig. 4,
Fig. 6 a perspective view of the drive spring of the device in Fig. 2,
Figs. 7a, b perspective views of the button and the number sleeve of the device in Fig. 2,
Fig. 8 a perspective view of parts of the drive mechanism of the device in Fig. 2,
Figs. 9a, b perspective views of the drive sleeve and the clutch plate of the device in Fig. 2,
Figs. 10a, b a dose setting sequence of the device in Fig. 2 in a side view,
Fig. 11 a perspective view of the button and the housing of the device in Fig.2,
Fig. 12 the device in Fig. 2 in a cut view,
Figs. 13a,b interaction between the drive sleeve and the number sleeve of the device in Fig. 2.
Fig. 14 shows a schematic illustration of one example of a dose limiting mechanism for use or integration with the injection device, wherein a first elongated component is located in a first longitudinal position,
Fig. 15 shows the dose limiting mechanism according to Fig. 14 with the first elongated component located in a second longitudinal position and
Fig. 16 shows the dose limiting mechanism of Figs. 14 and 15 after the first elongated component has returned from the second longitudinal position to the first longitudinal position,
Fig. 17 is illustrative of an example of the dose limiting mechanism, wherein an end stop is provided on the second elongated component,
Fig. 18 is illustrative of another example of the dose limiting mechanism, wherein an end stop is provided on the first elongated component,
Fig. 19 illustrates another example of the dose limiting mechanism, wherein the first elongated component is provided by a drive sleeve of the injection device,
Fig. 20 shows a schematic illustration of the second elongated component of the dose limiting mechanism implemented as a number sleeve of the injection device,
Fig. 21 is a perspective illustration of one example of a dose limiter to cooperate with the first and second elongated components as illustrated in Figs. 19 and 20,
Fig. 22 shows a portion of a longitudinal cross-section through a portion of the dose limiter of Figs. 21 and
Fig. 23 shows a portion of a transverse cross-section through the dose limiter of Fig. 21.

### Detailed description

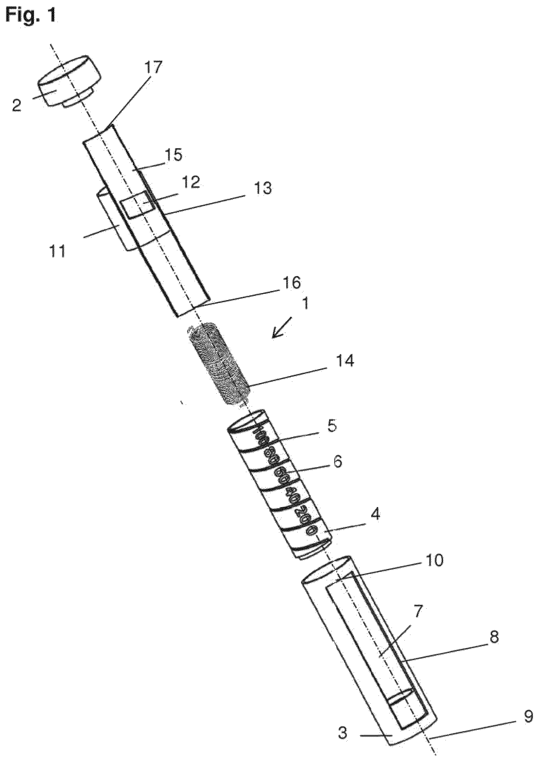

Figure 1 shows an exploded view of a first embodiment of the injection device 1 with its components which are a dose dial 2 in the form of a dial button, a housing or body 3, a dose scale drum or number sleeve 4 which has an outer thread 5 on its outer peripheral surface extending in a helical pattern from a distal end to a proximal end. The scale drum 4 carries indicia 6 which are printed on the scale drum. The indicia 6 are helically provided on the scale drum 4.

The housing or body 3 has an elongated window or aperture 7 of rectangular shape with two longitudinal borders 8 extending parallel to the longitudinal axis 9 of the injection device and two radial borders 10 perpendicular to the longitudinal axis 9. Through the window 7, the user can inspect the drum scale 4.

The dose dial 2 is axially retained in the housing 3 and the scale drum 4 is directly coupled to the dial button 2 to follow rotation of the dial button 2 such that when a user rotates the dial button 2 to select a dose, the scale drum 4 rotates together with the dial button 2. The dial button 2 and the scale drum 4 are arranged such that they both rotate without any axial displacement. The dose dial 2 also has the function of a dose or trigger button. The connection between the dial button 2 and the scale drum 4 can be made through a releasable coupling such that when the set dose is injected, the dial button 2 does not necessarily rotate back with the scale drum 4.

The external helical thread 5 of the scale drum 4 is engaged by a corresponding male thread of a sliding element 11. The sliding element 11 has a tubular section and a window or sliding window 12, wherein on two sides of the window 12 in axial direction, the male thread for engaging the helical thread 5 of the scale drum 4 is formed. In a further embodiment of the device shown, the inner surface of the sliding element 11 is in sliding contact with the outer surface of the scale drum between adjacent thread turns of the scale drum.

The inner surface of the housing 3 is provided with longitudinal bars that guide the sliding element 11 in axial direction but prevent relative rotation between the sliding element 11 and the housing 3. The longitudinal bars engage longitudinal recesses 13 on the outer surface of the sliding element 11. Due to this engagement in combination with the engagement between the threads of the housing 3 and the sliding element 11, the sliding element 11 moves axially whenever the scale drum 4 is rotated. The axial movement of the sliding element 11 and thus of the sliding window 12 relative to the longitudinal window 7 in the housing 3 is coordinated with the helical pattern of the indicia 6 printed on the scale drum 4 such that only one indicia 6 is present in the longitudinal window 7 and the sliding window 12 at the same time.

A drive spring 14 is connected to the scale drum 4 with one end and to the housing 3 with another end such that relative rotation between the scale drum 4 and the housing charges 3 the drive spring.

The axial length of the sliding element 11 is sufficient to cover the visible part of the helical track 5 of the drum scale 4 in order to fully prevent the user from viewing the indicia 6 not in sight through the sliding window 12. For that purpose, the sliding element 1 1 has an extension 15 that extends in the axial direction wherein the distal end 16 and proximal end 17 of the sliding element 1 1 is formed such that is does not collide with the borders 10 of the window 7. For that purpose, the distal border 10 may have a receiving section for receiving the extension such that the window 12 of the sliding element 1 1 can be placed over every single number on the scale drum 4.

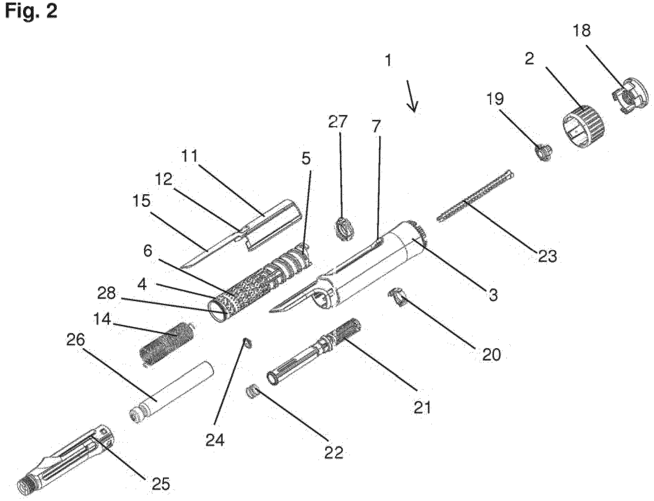

Figure 2 shows an exploded view of the components of a further embodiment of the injection device.

The device 1 comprises a dose dial 2 in the form of a dial grip, a housing and/or housing body 3 with an elongated window 7, a dose scale drum in the form of a number sleeve 4 which has an outer thread 5 on its outer peripheral surface extending in a helical pattern from a distal end to a proximal end. The number sleeve 4 carries indicia 6 which are printed on the scale drum. The indicia 6 are on the scale drum 4 in a helical pattern. The device further comprises a trigger button 18, a sliding element 11 configured as a gauge component with a sliding window 12, a clutch plate 19, a last dose nut 20, a drive sleeve 21, a clutch spring 22, a lead screw 23, a bearing 24 provided at a distal end of the lead screw 23, a drive spring 14 in the form of a torsion spring, a cartridge holder 25 that can be attached to the distal end of the housing 3 and that receives a cartridge 26 which is filled with a medicament and which has a bung or piston (not shown) inside wherein when the bearing 24 is moved in distal direction, the bearing displaces the bung such that medicament is dispensed from the cartridge 26 when a dispense interface such as a double ended needle cannula is attached to the distal end of the cartridge. The number sleeve 4 comprises an upper number sleeve part 27 referred to a number sleeve upper and a lower number sleeve part 28 referred to as number sleeve lower. In contrast to the embodiment in figure 1, the dose dial 2 and the button 18 are separate individual components. All components are located concentrically about a common principal longitudinal axis of the mechanism. The body 3 may also be a body element that it fixed to an outer housing or casing.

The button 18 is permanently splined to the dose dial 2. It is also splined to the number sleeve upper 28 when the button 18 is not pressed, but this spline interface is disconnected when the button 18 is pressed. When the button 18 is pressed, splines on the button 18 engage with splines on the housing 3 preventing rotation of the button 18 (and hence the dose dial 2) during dispense. These splines disengage when the button 18 is released, allowing a dose to be dialed. The dose dial 2 is axially constrained to the housing 3. It is rotationally constrained, via the splined interface to the button 18. The number sleeve lower 28 is rigidly fixed to the number sleeve upper 27 during assembly to form the number sleeve 4 and is a separate component to simplify number sleeve 4 mould tooling and assembly. This sub assembly is constrained to the housing 3 by holding elements (not shown) towards the distal end to allow rotation but not translation. The number sleeve lower 28 is marked with indices in the form of a sequence of numbers, which are visible through the window 12 of the sliding element 1 1 and the window 7 in the housing 3 to denote the dialed dose of medicament.

The clutch plate 19 is splined to the number sleeve 4. It is also coupled to the drive sleeve 21 via a ratchet interface. The ratchet provides a detented position between the number sleeve 4 and the drive sleeve 21 corresponding to each dose unit and engages different ramped tooth angles during clockwise and anti-clockwise relative rotation. The sliding element 11 is constrained to prevent rotation but allow translation relative to the housing 3 via a splined interface. The sliding element 11 has a helical feature on its inner surface which engages with the helical thread 5 cut in the number sleeve 4 such that rotation of the number sleeve 4 causes axial translation of the sliding element 11. This helical feature on the sliding element 11 also creates stop abutments against the end of the helical cut in the number sleeve 4 to limit the minimum and maximum dose that can be set.

In Figs. 1 - 13b a last dose nut 20 is located between the number sleeve 4 and the drive sleeve 21. It is rotationally constrained to the number sleeve 4 via a splined interface. It moves along a helical path relative to the drive sleeve 21 via a threaded interface when relative rotation occurs between the number sleeve 4 and drive sleeve 21. The drive sleeve 21 extends from the interface with the clutch plate 19 to the contact with the clutch spring 22. A splined tooth interface with the number sleeve 4 is not engaged during dialing, but engages when the button 18 is pressed, preventing relative rotation between the drive sleeve 21 and number sleeve 4 during dispense. A further splined tooth interface with the housing 3 prevents rotation of the drive sleeve 21 during dose setting. When the button 18 is pressed, the drive sleeve 21 and the housing 3 disengage allowing the drive sleeve 21 to rotate.

The helical drive spring 14 is charged and stores energy during dose setting by the action of the user rotating the dose dial 2. The spring energy is stored until the mechanism is triggered for dispense at which point the energy stored is used to deliver the medicament from the cartridge to the user. The drive spring 14 is attached at one end to the housing 3 and at the other end to the number sleeve 4. The drive spring 14 is pre-wound upon assembly, such that it applies a torque to the number sleeve 4 when the mechanism is at zero units dialed. The action of rotating the dose dial 2 to set a dose, rotates the number sleeve 4 relative to the housing 3 and charges the drive spring 14 further.

The lead screw 23 is rotationally constrained to the drive sleeve 21 via a splined interface. When rotated, the lead screw 23 is forced to move axially relative to the drive sleeve 21, through a threaded interface with the housing 3 (not shown). The bearing 24 is axially constrained to the lead screw 23 and acts on a bung within the liquid medicament cartridge 26. The axial position of the drive sleeve 21, clutch plate 19 and button 18 is defined by the action of the clutch spring 22, which applies a force on the drive sleeve 21 in the proximal direction. This spring force is reacted via the drive sleeve 21, clutch plate 19 and button 18, and when 'at rest' it is further reacted through the dose dial 2 to the housing 3. The spring force ensures that the ratchet interface is always engaged. In the 'at rest' position, it also ensures that the button splines are engaged with the number sleeve 4 and that the drive sleeve teeth are engaged with the housing 3. The housing 3 provides location for the liquid medication cartridge and cartridge holder 25, windows for viewing the dose number and the sliding element, and a feature on its external surface to axially retain the dose dial 2 (not shown). A removable cap fits over the cartridge holder 25 and is retained via clip features on the housing 3.

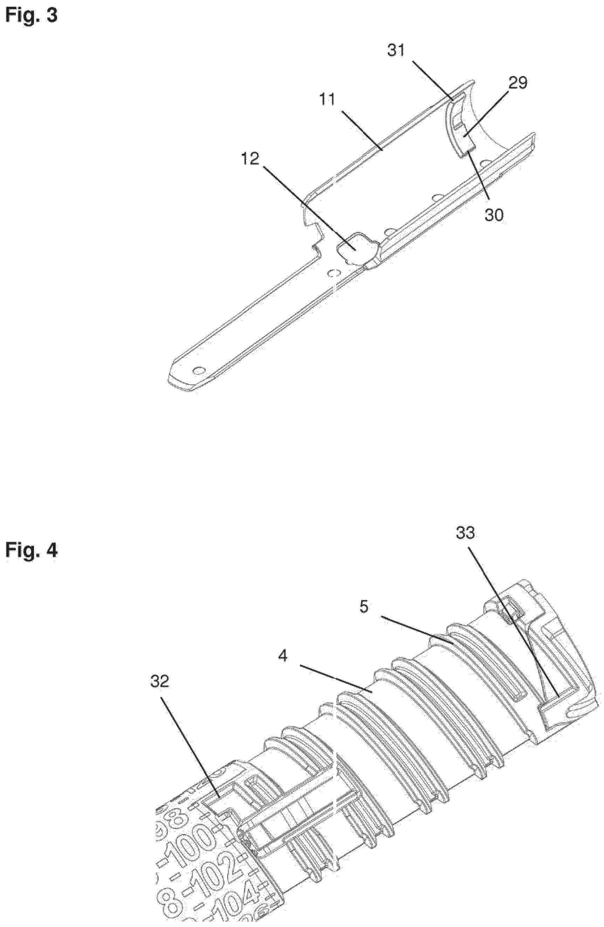

Figure 3 shows the inside of the sliding element 11 with the window 12 and the male thread feature 29 on the inner surface of the sliding element 11 that engages the outer thread 5 on the number sleeve 4 (see figure 4). The thread feature 29 has a zero dose abutment 30 and a maximum dose abutment 31. As shown in figure 4, the outer thread 5 has a zero dose abutment 32 at one end of the thread 5 and a maximum dose abutment 33 at the other end of the thread 5 so that any dose size can be selected between zero and a pre-defined maximum, in increments to suit the medicament and user profile. The drive spring 14, which has a number of pre-wound turns applied to it during assembly of the device, applies a torque to the number sleeve 4 and is prevented from rotating by the zero dose abutment.

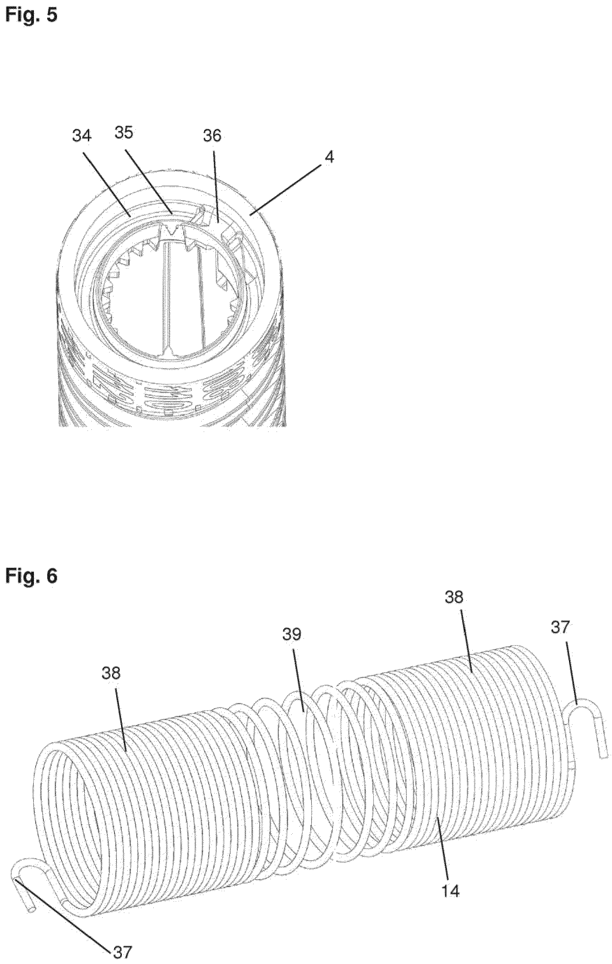

As shown in figure 5, the inner surface of the number sleeve 4 has a lead-in 34 followed by a groove 35 and an anchor point 36. Automated assembly of the drive spring 14 into the number sleeve is achieved by incorporating the large lead-in 34 and the groove feature 35. As the drive spring 14 is rotated during assembly, a hook end form 37 at the one end of the drive spring 14 (see figure 6) locates in the groove feature 35 before engaging the anchor point 36 in the number sleeve 4.

As shown in figure 6, the drive spring 14 is formed from a helical wire with at least two different pitches. Both ends are formed from 'closed' coils 38, i.e. the pitch equals the wire diameter and each coil contacts the adjacent coil. The central portion has 'open' coils 39, i.e. the coils do not contact each other. This has the following advantages. When a dose is set, the drive spring 14 is charged. **If** all the coils were closed, winding up the spring would increase the length of the spring by one wire diameter for each turn, and so the hook ends would no longer be aligned with their anchor points on the housing and number sleeve. The open coils allow the spring to compress to accommodate the additional turns of wire, without increasing the total length of the spring. Further, the open coils 39 allow the spring to be compressed during assembly. The spring is manufactured longer than the space available in the device. It is then compressed during assembly, ensuring that the axial positions of the hook ends are better aligned with their anchor points on the housing and the number sleeve. Also, it is easier to manufacture the spring to a specified length if most of the coils are closed, as the length of these coils is only a function of the wire diameter. Moreover, following assembly, compression in the spring biases the number sleeve axially relative to the housing in a consistent direction, reducing the effects of geometric tolerances. Further, the addition of closed coils at each end makes the springs less prone to tangling with each other when they are stored together between manufacture and assembly and closed coils at the ends provide a flat surface for contact with the housing and the number sleeve.

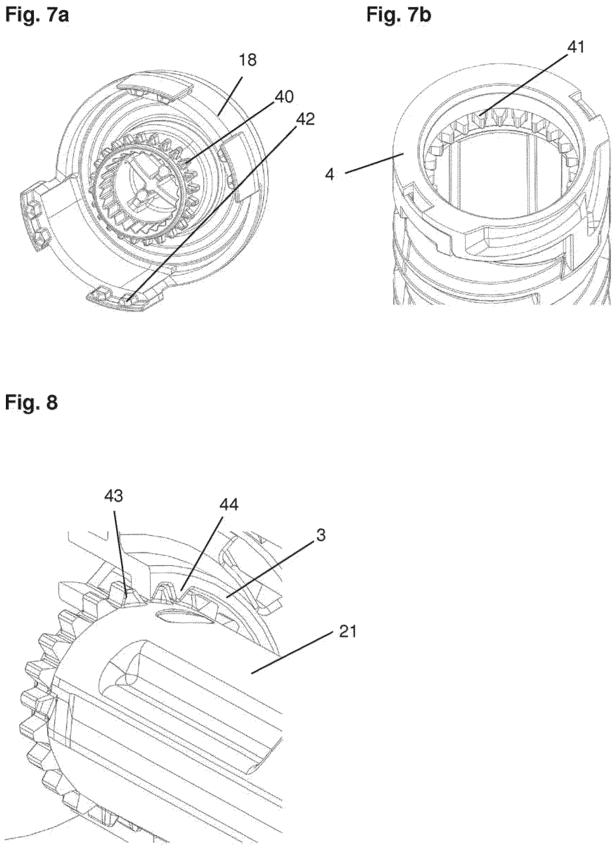

For selecting a dose, the user rotates the dial grip 2 clockwise. As shown in figures 7a and 7b, the button has inner splines 40 for engaging corresponding splines 41 on the upper part of number sleeve 4 to create a splined interface 40/41. The dial grip is splined to the button 18, wherein the button 18 has a further set of splines 42 for engagement with corresponding splines of the housing 3. During dose selection, rotation of the dial grip is transferred to the button 18. The button 18 is in turn splined to the number sleeve upper (during dose selection only) via the splines 40. The number sleeve upper is permanently fixed to the number sleeve lower to form the number sleeve 4. Therefore, rotation of the dial grip 2 generates an identical rotation in the number sleeve 4. Rotation of the number sleeve 4 causes charging of the drive spring, increasing the energy stored within it. As the number sleeve 4 rotates, the sliding element 1 1 translates axially due to its threaded engagement with the number sleeve 4 thereby showing the value of the dialed dose.

As shown in figure 8, the drive sleeve 21 has splines 43 for engaging corresponding splines 44 formed on the inside of the housing 3 to create a splined interface 43/44. The drive sleeve 21 is prevented from rotating as the dose is set and the number sleeve is rotated, due to the engagement of its splined teeth 43 with the teeth 44 of the housing 3. Relative rotation therefore occurs between the clutch plate that is driven by the number sleeve and the drive sleeve via the ratchet interface.

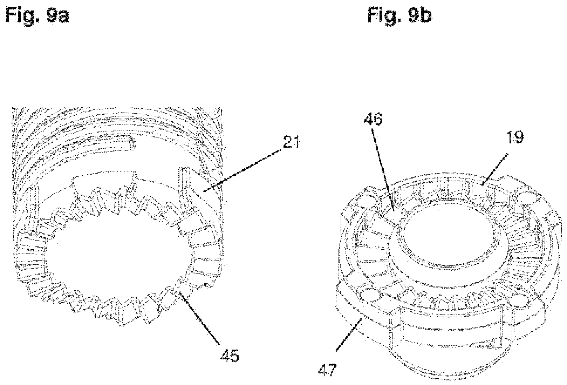

As shown in figure 9a and 9b, an end surface of the drive sleeve 21 is provided with angled teeth 45 to form a ratchet interface 45/46 with angled teeth 46 on the clutch plate 19. On the outer circumference of the clutch plate 19, splined teeth 47 for engaging a corresponding groove on the number sleeve are formed. The user torque required to rotate the dial grip is a sum of the torque required to wind up the drive spring, and the torque required to overhaul the ratchet feature 45/46. The clutch spring is designed to provide an axial force to the ratchet feature 45/46 and to bias the clutch plate 19 onto the drive sleeve 21. This axial load acts to maintain the ratchet teeth engagement of the clutch plate 19 and the drive sleeve 21. The torque required to overhaul the ratchet in the dose set direction is a function of the axial load applied by the clutch spring, the clockwise ramp angle of the ratchet, the friction coefficient between the mating surfaces and the mean radius of the ratchet features. As the user rotates the dial grip sufficiently to increment the mechanism by 1 increment, the number sleeve 14 rotates relative to the drive sleeve 21 by 1 ratchet tooth. At this point the ratchet teeth re-engage into the next detented position. An audible click is generated by the ratchet re-engagement, and tactile feedback is given by the change in torque input required.

With no user torque applied to the dial grip 21, the number sleeve 4 is prevented from rotating back under the torque applied by the drive spring 14, solely by the ratchet engagement 45/46 between the clutch plate 19 and the drive sleeve 21. The torque necessary to overhaul the ratchet in the anti-clockwise direction is a function of the axial load applied by the clutch spring 22, the anti-clockwise ramp angle of the ratchet 45/46, the friction coefficient between the mating surfaces and the mean radius of the ratchet features. The torque necessary to overhaul the ratchet must be greater than the torque applied to the number sleeve 4 (and hence clutch plate 19) by the drive spring 14. The ratchet ramp angle is therefore increased in the anticlockwise direction to ensure this is the case whilst ensuring the dial-up torque is as low as possible.

The user may choose to increase the selected dose by continuing to rotate the dial grip in the clockwise direction. The process of overhauling the ratchet interfaces between the number sleeve 4 and drive sleeve 21 is repeated for each dose increment. Additional energy is stored within the drive spring 14 for each dose increment and audible and tactile feedback is provided for each increment dialled by the re-engagement of the ratchet teeth. The torque required to rotate the dial grip 2 increases as the torque required to wind up the drive spring 14 increases. The torque required to overhaul the ratchet in the anti-clockwise direction must therefore be greater than the torque applied to the number sleeve 4 by the drive spring 14 when the maximum dose has been reached.

If the user continues to increase the selected dose until the maximum dose limit is reached, the number sleeve 4 engages with its maximum dose abutment on the sliding element (see figure 3 and 4). This prevents further rotation of the number sleeve 4, clutch plate 19 and dial grip 2.

The last dose nut 20 is splined to the number sleeve 4 while the last dose nut 20 is threaded to the drive sleeve such that relative rotation of the number sleeve and the drive sleeve during dose setting also causes the last dose nut to travel along its threaded path towards a last dose abutment on the drive sleeve. Depending on how many increments have already been delivered by the mechanism, during selection of a dose, the last dose nut may contact its last dose abutment with the drive sleeve. The abutment prevents further relative rotation between the number sleeve 4 and the drive sleeve 21 and therefore limits the dose that can be selected. The position of the last dose nut is determined by the total number of relative rotations between the number sleeve 4 and the drive sleeve 21, which have occurred each time the user sets a dose.

When a dose has been set, the user is able to deselect any number of increments from this dose. Deselecting a dose is achieved by the user rotating the dial grip 2 anti-clockwise. The torque applied to the dial grip 2 by the user is sufficient, when combined with the torque applied by the drive spring 14, to overhaul the ratchet between the clutch plate 19 and the drive sleeve 21 in the anti-clockwise direction. When the ratchet 45/46 is overhauled, anti-clockwise rotation occurs in the number sleeve 4 (via the clutch plate 19), which returns the number sleeve 4 towards the zero dose position, and unwinds the drive spring 14. The relative rotation between the number sleeve 4 and drive sleeve 21 causes the last dose nut to return along its helical path, away from the last dose abutment.

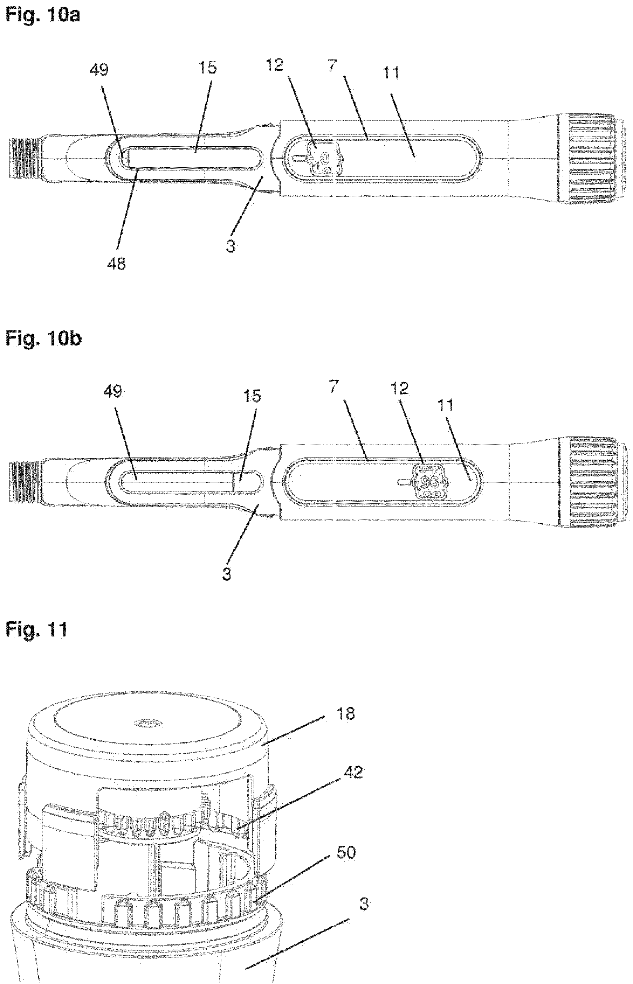

As shown in figures 10a and 10b, the sliding element 11 has flanges or extensions on either side of the window area which cover the numbers printed on the number sleeve adjacent to the dialed dose to ensure only the set dose number is made visible to the user. The device includes a visual feedback feature in addition to the discrete dose number display typical on devices of this type. The distal end of the sliding element 11 has the extension 15 (see figure 2) that creates a sliding scale through a small window 48 in the housing 3. As a dose is set by the user, the sliding element 11 translates axially, the distance moved proportional to the magnitude of the dose set. This feature gives clear feedback to the user regarding the approximate size of the dose set. The dispense speed of an auto-injector mechanism may be higher than for a manual injector device, so it may not be possible to read the numerical dose display during dispense. The sliding element 1 1 provides feedback to the user during dispense regarding dispense progress without the need to read the dose number itself.

The window 48 may be formed by an opaque element on the sliding element 11 revealing a contrasting coloured component 49 underneath. Alternatively, the revealable element 49 may be printed with coarse dose numbers or other indices to provide more precise resolution. In addition, this display simulates a syringe action during dose set and dispense.

To reduce dust ingress and prevent the user from touching moving parts, the viewing openings 7 and 48 in the housing 3 are covered by translucent windows. These windows may be separate components, but in this embodiment they are incorporated into the housing 3 using 'twin-shot' moulding technology. A first shot of translucent material forms the internal features and the windows, and then a 'second shot' of opaque material forms the outer cover of the housing 3.

Delivery of a dose is initiated by the user depressing the button axially. When the button 18 (see figures 7a and 7b) is depressed, the splines 40 and 41 between the button 18 and the number sleeve 4 disengage, rotationally disconnecting the button 18 and dial grip 21 from the delivery mechanism.

As shown in figure 11, the splines 42 on the button 18 engage with splines 50 on the housing 3 preventing rotation of the button 18 (and hence the dial grip 21) during dispense. As the button 18 is stationary during dispense, it can be used in a dispense clicker mechanism. A stop feature in the housing 3 limits axial travel of the button 18 and reacts any axial abuse loads applied by the user, reducing the risk of damaging internal components.

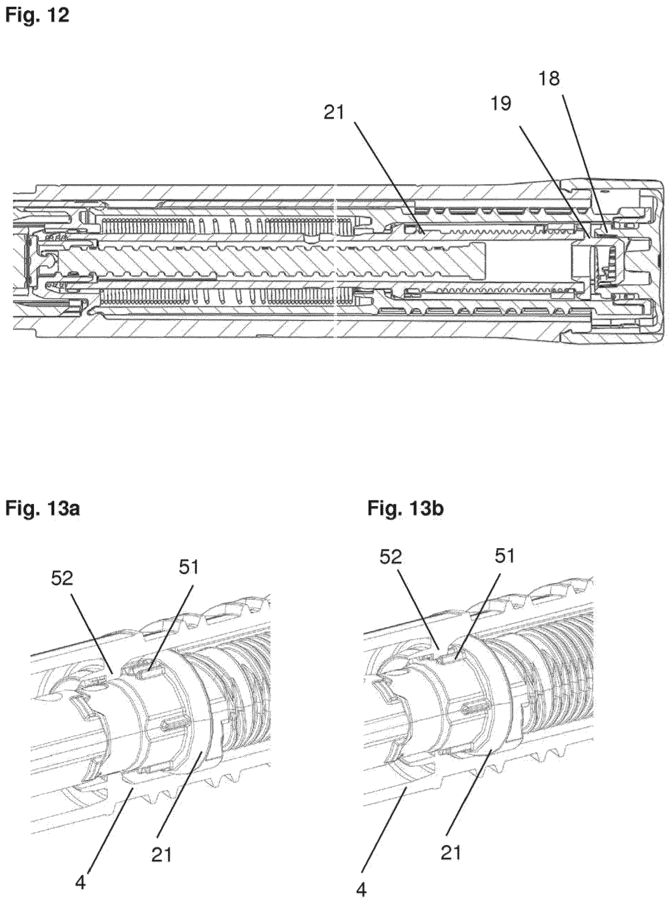

As shown in figure 12, the clutch plate 19 arranged between the drive sleeve 21 and the button 18 is moved axially by the button and the drive sleeve 21 is moved axially by the clutch plate 19. As shown in figure 13a and 13b, the axial displacement of the drive sleeve 21 engages splines 51 on the drive sleeve 21 with splines 52 on the number sleeve 4 so that a splined tooth interface 51/52 is formed preventing relative rotation between the drive sleeve 21 and number sleeve 4 during dispense. The splined tooth interface 43/44 (figure 8) between the drive sleeve 21 and the housing 3 disengages, so that the drive sleeve 21 can now rotate relative to the housing 3 and is driven by the drive spring via the number sleeve 4, and clutch plate 19. Rotation of the drive sleeve 21 causes the lead screw 23 to rotate due to their splined engagement, and the lead screw 23 then advances due to its threaded engagement to the housing 3. The number sleeve 4 rotation also causes the sliding element to traverse axially back to its zero position whereby the zero dose abutment (figure 3 and figure 4) stops the mechanism.

It is possible to angle the spline teeth on either the drive sleeve 21 or the housing 3, so that when the zero dose abutment 30 stops rotation of the number sleeve 4 and hence the drive sleeve 21 at the end of the dose and the button 18 is released the spline teeth between the drive sleeve 21 and the housing 3 rotate the drive sleeve 21 backwards by a small amount and hence move the lead screw 23 axially back away from the bung and rotates the number sleeve lower 28 from the zero dose stop position. This helps to prevent possible weepage.

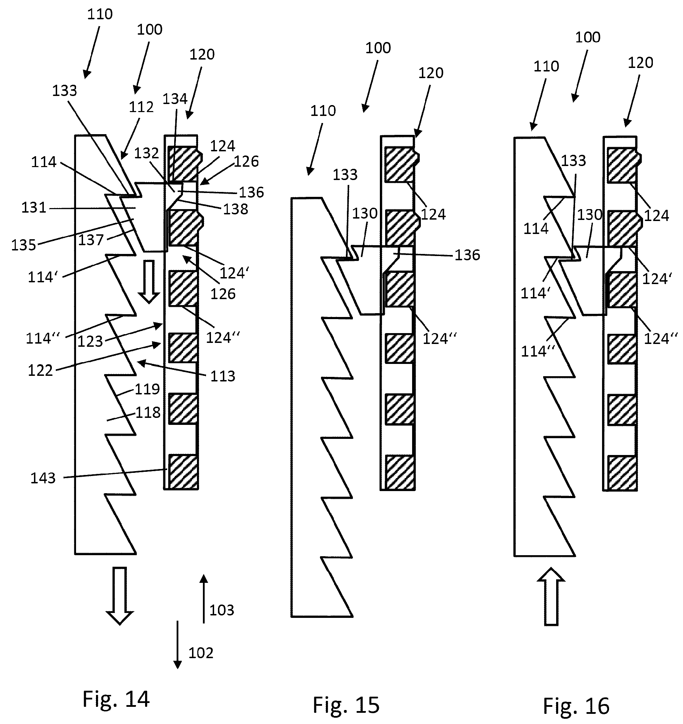

In Figs. 14-18 the principle of the dose limiting mechanism 100 according to the present disclosure and configured for an injection device 1, such as e.g. shown and described in connection with Figs. 1 - 13b, is schematically illustrated. The dose limiting mechanism 100 is operable to limit a total number of doses dispensable by the injection device 1. The dose limiting mechanism 100 comprises a first elongated component 110 extending in a longitudinal direction and comprising a first profiled structure 112. The dose limiting mechanism 100 further comprises a second elongated component 120 coaxial or extending parallel to the first elongated component 110. The second elongated component 120 comprises a second profiled structure 122 facing towards the first profiled structure 112. There is further provided a dose limiter 130. The dose limiter 130 is arranged between the first elongated component 110 and the second elongated component 120. The dose limiter 130 comprises a first profile section 131 and a second profile section 132. The first profile section 131 faces towards the first profiled structure 112. It is configured to engage, i.e. to mechanically engage with the first profiled structure 112 of the first elongated component 110. Likewise, the second profile section 132 of the dose limiter 130 is configured to engage, i.e. to mechanically engage with the second profiled structure 122 of the second elongated component 120.

The mutual engagement of first and second profiled structures 112, 122 with respective first and second profile sections 131, 132 is such, that a longitudinal movement, e.g. a longitudinal sliding movement of the first elongated component 110 relative to the second elongated component 120 is transferred to the dose limiter 130. Here, the dose limiter 130 is allowed to move in distal direction 102 relative to the second elongated component 120. The dose limiter 130 is pushable or draggable by the first elongated component in distal direction 102. It is hindered to move proximally relative to the first elongated component 110. In this way a distally directed movement of the first elongated component is 110 transferred to the dose limiter 130. This is immediately apparent from a comparison of Figs. 14 and 15.

The second profile section 132 of the dose limiter 130 is further engageable with the second profiled structure 122 to retain a longitudinal position of the dose limiter 130 relative to the second component 120 when the first component should be subject to a longitudinal movement in a proximal direction 103 relative to the second elongated component 120. In other words, the mutual engagement of the second profile section 132 with the second profiled structure 122 is such that the dose limiter 130 is always allowed to move distally relative to the second elongated component 120 but is hindered or blocked from moving in proximal direction relative to the second elongated component 120.

Likewise, the first elongated component 110 is allowed to move in proximal direction 103 relative to the dose limiter 130 but is blocked or hindered to move in distal direction 102 relative to the dose limiter 130. Any distally directed displacement of the first elongated component 110 relative to the second elongated component 120 transfers to the dose limiter 130. As the first elongated component 110 is subject to a movement in distal direction 102 the dose limiter 130 has to follow this distally directed movement. It is pushed in distal direction 102 accordingly.

With the presently illustrated example the first profiled structure 112 of the first elongated component 110 comprises a regular sawtooth profile 113. The saw tooth profile 113 comprises a number of teeth 118 adjacently arranged in longitudinal direction. The teeth 118 comprise a rather steep flank forming or constituting a stop face 114 and facing in distal direction 102. The teeth 118 further comprise a somewhat beveled flank 119 facing towards the second elongated component 120.

The dose limiter 130 comprises a correspondingly shaped first profile section 131. The first profile section 131 comprises a counter stop face 133 facing in the proximal direction 103 and being configured to engage with the distally facing stop faces 114 of the teeth 118 of the profiled structure 112 of the first elongated component 110.

The first profile section 132 of the dose limiter 130 is provided on a projection or protrusion 135 extending towards the first elongated component 110. The protrusion 135 is shaped to enter transverse or radial recesses formed by or adjacent to the stop faces 114, 114', 114" of the teeth 118.

The second profile section 132 of the dose limiter 130 facing towards the second elongated component 120 also comprises a transverse or radial protrusion 136 protruding towards the second elongated component 120. The protrusion 136 is shaped and configured to engage with the retaining faces 124, 124', 124" of the second profiled structure 132. The retaining faces 124, 124', 124" are provided on a proximal side of respective recesses 126 arranged in consecutive and regular order along the longitudinal direction on that side of the second elongated component 120 that faces towards the first elongated component 110.

As indicated in Figs. 14-16 the proximally facing counter stop face 134, e.g. provided at a proximal end of the protrusion 136 is in proximal abutment with the distally facing retaining face 124 of a first recess 126 of the second profiled structure 132

When starting from an initial configuration as illustrated in Fig. 14, in which the first elongated component 110 is in a first longitudinal position and as the first elongated component 110 is subject to a movement relative to the second elongated component 120 along the distal direction 102 the stop face 114 at the first profiled structure 112 applies a distally directed force onto the dose limiter 130. A distally facing side of the protrusion 136 comprises a beveled edge 138 by way of which the dose limiter 130 is allowed to smoothly slide in distal direction 102 out of the pocket or recess 126 until the second profile section 132 engages with a consecutive retaining face 124' of a subsequent recess 126' of the second profiled structure 122 as illustrated in Fig. 15.

Here, and compared to the configuration as illustrated in Fig. 14 the first elongated component 110 has reached the second longitudinal position. In this position and since the proximally facing counter stop face 134 of the dose limiter 130 is in longitudinal and proximal engagement with the distally facing second retaining face 124' of the second profiled structure 122 the dose limiter 130 is hindered to return into the proximal position as illustrated in Fig. 14. Rather and as the first elongated component 110 is subject to a return movement towards the proximal direction 103, which motion may be e.g. induced or governed by a trigger spring the sawtooth profile 113 of the first profiled structure 112 is allowed to slide along a first beveled side edge 137 of the dose limiter 130 facing towards the first elongated component 110. Then and as illustrated in Fig. 16 the first elongated component 110 is allowed to return proximally into the first longitudinal position in which a consecutive second stop face 114' of a consecutive tooth 118' gets in axial abutment with the first profile section 131 of the dose limiter 130.

In effect, the proximally facing first counter stop face 133 of the dose limiter 130 may face towards the distally facing stop face 114' of the second toothed 118' of the first elongated component 110. Here, it is not required that the first counter stop face 133 and the stop face 114' get in direct longitudinal abutment. There may be provided an axial or longitudinal gap which will be closed when the first elongated component 110 should be subject to another movement in distal direction 102, e.g. during a subsequent dose dispensing procedure.

Even though not particularly illustrated the dose limiter 130 as well as first and second profiled structures 112, 122 may be shaped and arranged in such a way, that mutually corresponding beveled edges or side faces are allowed to slide in longitudinal direction relative to each other. In detail, and when in the configuration of Fig. 15 the flank portion 119 of a tooth 118' of the first elongated component 110 is allowed to slide in proximal direction 103 along the beveled edge 137 of the dose limiter 130 while the dose limiter 130 is hindered from moving proximally by the engagement or abutment of the second profile section 132 with the second profiled structure 122.

If the presently illustrated example the first profiled structure 112 is illustrated as a sawtooth profile 113 and the second profiled structure 122 is implemented as a recessed profile 123 featuring numerous recesses 126 equidistantly arranged along the longitudinal direction. Generally, the dose limiter 130 can be implemented and configured in many different ways. There are configurations conceivable, wherein e.g. the dose limiter 130 comprises a recessed structure and wherein the second profiled structure 122 comprises a sawtooth profile. With some other examples one of the first profile section 131 and the first profiled structure 112 comprises a protrusion or a protruding sawtooth profile whereas the other one of the first profile section 131 and the first profiled structure 112 comprises a correspondingly shaped recessed profile.

The dose limiting mechanism 100 as illustrated in the sequence of Figs. 14-18 is generally applicable to a large variety of injection devices, wherein a first elongated component 110 is subject to a well-defined stepwise distally directed longitudinal movement relative to the second elongated component 120 during or for initiating a dose dispensing procedure and wherein the first elongated component is subject to a return motion in proximal direction 103 at the end of the dose dispensing procedure. Each time the first elongated component 110 is subject to the distally directed movement, e.g. initiated by depressing of a trigger or trigger button 18 of the injection device 1 the dose limiter 130 is moved one step further. The step size is governed by the periodicity or longitudinal size of the first and second profiled structures 112, 122 as well as by the longitudinal distance between the first and the second longitudinal positions of the first elongated component 110.

Typically, the total number of teeth or the total number of discrete stop faces or retaining faces provided on the first and/or second profiled structures defines the total number of doses that can be injected or dispensed by the injection device 1.

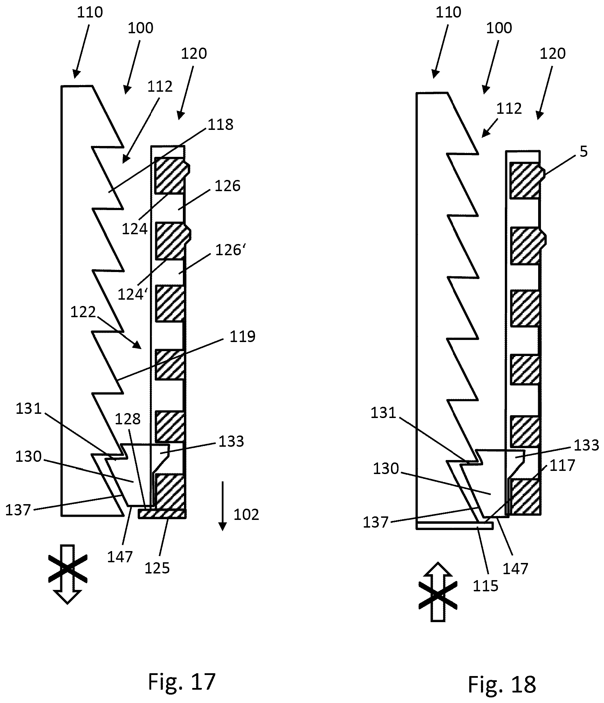

For effectively limiting or disabling further use of the injection device after termination or processing of an allowable number of dose dispensing procedures there is provided at least one end stop 115, 125 on at least one of the first elongated component 110 and the second elongated component 120. One example of an end stop 125 is illustrated in Fig. 17. There, the end stop 125 is provided at or near a distal end of the second elongated component 120. The end stop 125 protrudes radially inwardly in transverse direction from the longitudinal orientation of the second elongated component 120 and towards the first elongated component 110. The end stop 125 protrudes inwardly into the gap or interstice between the first and second elongated components 110, 120.

The end stop 125 comprises a proximally facing abutment face 128 configured to abut with a correspondingly shaped distally facing abutment face 147 of the dose limiter 130. As soon as a stop configuration as illustrated in Fig. 17 has been reached, in which the abutment face 147 is in engagement with the abutment faces 128 any further distally directed movement of the dose limiter 130 initiated through the respective distally directed movement of the first elongated component 110 is effectively blocked. In this way, the first elongated component 110 cannot be moved any further in distal direction 102.

Typically, and when the first elongated component 110 is in axial abutment or axial engagement with a trigger or trigger button 18 of the injection device 1 a repeated depression of the trigger or trigger button 18 in distal direction 102 is effectively blocked and a further dose dispensing action cannot be performed with the injection device 1.

Another configuration of an end stop 115 is illustrated in Fig. 18. There, the end stop 115 is provided on the first elongated component 110. Also here, the end stop 115 protrudes inwardly into the gap or into the interstice between the first elongated component 110 and the second elongated component 120. The end stop 115 comprises a proximally facing abutment face 117 to engage with the distally facing abutment face 147 of the dose limiter 130. Here and when the first elongated component 110 is axially or longitudinally connected to the trigger or trigger button 18 of the injection device 1 the mutual abutment of the corresponding abutment faces 117, 147 prevents and locks a return motion of the first elongated component 110 in proximal direction 103 relative to the second elongated component 120 at the end of a final or last allowable dose dispensing procedure.

As a consequence, the trigger or trigger button 18 may be hindered to return into an initial position in which it can be depressed by a user. A release of the trigger or trigger button 18 may therefore not lead to a usual return motion of the trigger or trigger button in proximal direction. Moreover, a clutch between the dose dial and the second elongated component 120, e.g. implemented as a number sleeve, may permanently remain disengaged. In this way, a user will be also hindered to set or to dial a further dose of a medicament.

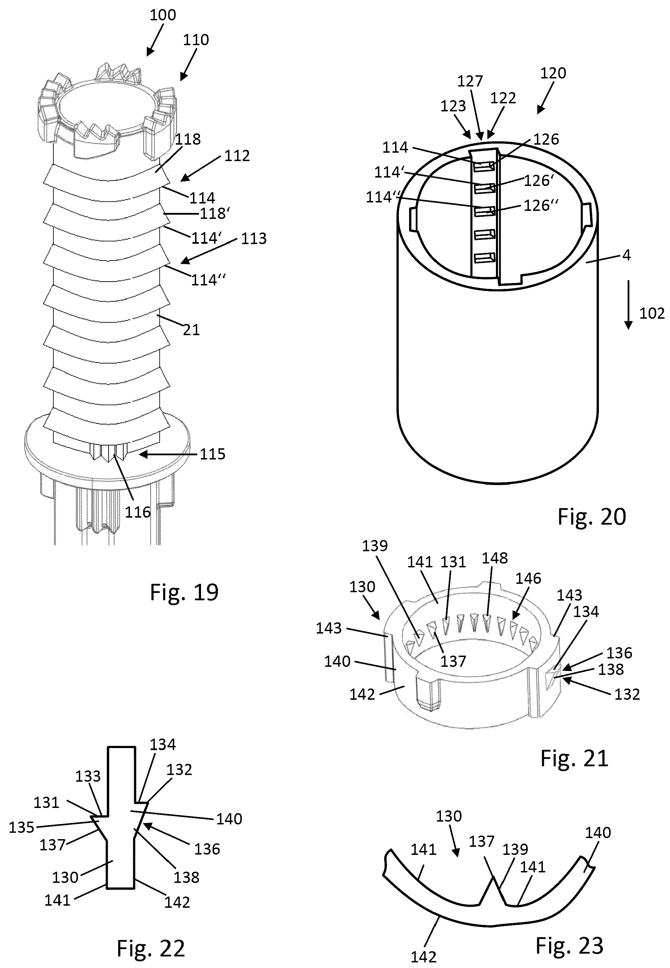

In the further illustrations of Figs. 19-23 the dose limiting mechanism 100 as explained above in connection with Figs. 14-18 is implemented with or integrated into the injection device 1 as described and illustrated with regard to Figs. 1 - 13b.

Here, the drive sleeve 21 is implemented as the first elongated component 110 and the number sleeve 4 is implemented as the second elongated component 120. The second elongated component 120 may be threadedly engaged with the housing 3. Respective thread or threaded sections are not particularly illustrated in Fig. 20. The outer thread 5 of an implementation of the second elongated component 120 as a number sleeve 4 is for instance illustrated in Fig. 18. Contrary to the example of Figs. 1 - 13b and when the first elongated component 110 is implemented as a drive sleeve 21 it is void of an external thread but comprises a sawtooth profile 113 featuring a sequence of distally facing annular shaped abutment faces 114, 114', 114" as indicated in Fig. 14.

With the example of Figs. 19 - 23 the dose limiter 130 is permanently rotationally locked to the second elongated component 120. For this the dose limiter 130 comprises at least one or numerous spline features 143 on an outside surface engaged with correspondingly shaped spline features 127 on the inside of the tubular shaped second elongated component 120. The dose limiter 130 is displaceable in longitudinal direction inside the second elongated component 120. As illustrated in Fig. 20 the recessed profile 123 is implemented on or in a bottom of the spline feature 127, which is provided in the sidewall of the second elongated component 120.

The spline feature 127 comprises an elongated groove on the inside of the sidewall of the tubular shaped second elongated component 120. The groove or spline feature 127 is further provided with numerous recesses 126, 126', 126", each of which providing a respective retaining face 124, 124', 124" facing in distal direction 102. The dose limiter 130 comprises an annular ring 140. On the outside surface 142 there are provided the spline features 143 to engage with the correspondingly shaped spline features 127 of the second elongated component 120. On the spline features 143 the dose limiter 130 comprises at least one radially outwardly extending protrusion 136 forming or constituting the second profile section 132 to engage with the second profiled structure 122 as provided by the recessed profile 123 of the second elongated component 120. As illustrated in Fig. 21 the protrusion 136 comprises a proximally facing counter stop face 134 to engage with the distally facing retaining faces 124, 124', 124" of the second profiled structure 122. Generally, and with other examples the second profile section 132 may be located elsewhere on the outside surface of the dose limiter 130. It may be arranged circumferentially offset from the spline feature 143.

On the inside surface 141 of the dose limiter, e.g. on the inside of the ring 140 there is provided a gear ring 146 that comprises numerous teeth 148. The teeth 148 each comprise or constitute the first profile section 131 of the limiter 130. The teeth 148 are arranged regularly along the inner circumference of the ring 140. Each one of the teeth 148 comprises a proximally facing first counter stop face 133 to engage with the sawtooth profile 113 as provided on the outside surface of the tubular shaped first elongated component 110. In detail, the first counter stop faces 133 of the numerous teeth 148 are configured to engage or to abut in longitudinal direction with the distally facing stop faces 114, 114', 114" of the first profiled structure 112. In this way, the dose limiter 130 can dragged or pushed in distal direction 102 by the first elongated component 110 relative to the second elongated component 120, namely by the annular shaped and distally facing stop face 114 of the first profiled structure 112. The stop face 114 gets in distally directed abutment with the first counter stop face 133 of the respective first profile section 131 of the dose limiter 130.

From Fig. 19 it is further apparent that the end stop 115 as provided on the first elongated component 110 comprises numerous teeth 116. The teeth 116 are configured to rotationally lock with the teeth 148 and hence with the gear ring 146 provided on the inside surface 141 of the dose limiter 130. During setting of a dose and when located proximally from the end stop 115 the dose limiter 130 is free to rotate relative to the first elongated component 110. Due to the splined engagement with the second elongated component 120 the dose limiter 130 rotates relative to the first elongated component 110 and hence relative to the drive sleeve 21 during setting of a dose.

The teeth 148 protruding radially inwardly from the inside surface 141 of the dose limiter 130 comprise oppositely located side flanks 139 as schematically illustrated in Figs. 21 and 23. The oppositely located side flanks 139 are somewhat symmetrical in circumferential direction. They merge with the beveled edge 137. As indicated in Fig. 21, the side flanks 139 are converging in distal direction 102. This facilitates a rather smooth engagement with the teeth 116 of the end stop 115 when the respective stop configuration has been reached.

During setting of a dose the drive sleeve 21 and hence the first elongated component 110 is rotationally locked to the housing 3 of the injection device 1 whereas the number sleeve 4 or the scale drum is subject to a rotation. Now and when the dose limiter 130 longitudinally aligns or longitudinally overlaps with the end stop 115, e.g. after a final or last dose dispensing action has terminated the gear ring 146 rotationally locks to the at least one locking tooth 116 provided on the outside surface of the first elongated component 110 or drive sleeve 21. Due to this rotational interlock the drive sleeve 21 will be rotationally locked to the number sleeve 4.

In this way and since the drive sleeve 21 is rotationally locked to the housing 3 the number sleeve 4 and hence the dose dial 12 cannot be rotated any further for setting of a dose. When reaching the end of dose configuration and when a predefined allowable number of doses has been dispensed by the injection device 1 the number sleeve 4 is rotationally locked to the drive sleeve 21 and any further use of the injection device 1 is effectively blocked and impeded.

### List of reference numbers

- 1: Injection device (drug delivery device)
- 2: dose dial / dial grip
- 3: housing / body
- 4: dose scale drum / number sleeve
- 5: outer thread
- 6: indicia
- 7: window
- 8: longitudinal border
- 9: longitudinal axis
- 10: radial border
- 11: sliding element
- 12: sliding window
- 13: recess
- 14: drive spring
- 15: extension
- 16: distal end of sliding element
- 17: proximal end of sliding element
- 18: trigger button
- 19: clutch plate
- 20: last dose nut
- 21: drive sleeve
- 22: clutch spring
- 23: lead screw
- 24: bearing
- 25: cartridge holder
- 26: cartridge
- 27: upper number sleeve part
- 28: lower number sleeve part
- 29: male thread feature
- 30: zero dose abutment of sliding element
- 31: maximum dose abutment of sliding element
- 32: zero dose abutment of number sleeve
- 33: maximum dose abutment of number sleeve
- 34: lead-in
- 35: groove
- 36: anchor
- 37: hook
- 38: closed coils
- 39: open coils
- 40: splines of button
- 41: splines of number sleeve
- 42: splines of button
- 43: splines of drive sleeve
- 44: splines of body
- 45: angled teeth
- 46: angled teeth
- 47: splines of clutch plate
- 48: window
- 49: revealable element
- 50: splines on body
- 51: splines on drive sleeve
- 52: splines on number sleeve
- 100: dose limiting mechanism
- 102: distal direction
- 103: proximal direction
- 110: elongated component
- 112: profiled structure
- 113: sawtooth profile
- 114: stop face
- 115: end stop
- 116: locking tooth
- 117: abutment face
- 118: tooth
- 119: flank portion
- 120: elongated component
- 122: profiled structure
- 123: recessed profile
- 124: retaining face
- 125: end stop
- 126: recess
- 127: spline feature
- 128: abutment face
- 130: dose limiter
- 131: profile section
- 132: profile section
- 133: counter stop face
- 134: counter stop face
- 135: protrusion
- 136: protrusion
- 137: beveled edge
- 138: beveled edge
- 139: flank
- 140: ring
- 141: inside surface
- 142: outside surface
- 143: spline feature
- 146: gear ring
- 147: abutment face
- 148: tooth

## Claims

1. A dose limiting mechanism (100) for an injection device (1), the dose limiting mechanism (100) being configured for arrangement inside a housing (3) of the injection device (1) and being operable to limit a total number of doses dispensable by the injection device, the dose limiting mechanism (100) comprising:
- a first elongated component (110) extending in a longitudinal direction and comprising a first profiled structure (112), wherein the first profiled structure (122) comprises a number of stop faces (114, 114', 114") consecutively arranged in longitudinal direction and facing in the distal direction,
- a second elongated component (120) coaxial or parallel to the first elongated component (110) and comprising a second profiled structure (122) facing towards the first profiled structure (112),
- a dose limiter (130) arranged between the first elongated component (110) and the second elongated component (120), the dose limiter (130) comprising a first profile section (131) and a second profile section (132),
- wherein the first profile section (131) being engagable with the first profiled structure (112) to transfer a longitudinal movement of the first elongated component (110) to the dose limiter (130) when the first elongated component (110) is subject to a longitudinal movement in a distal direction relative to the second elongated component (120) during dispensing of a dose, and
- wherein the second profile section (132) being engagable with the second profiled structure (122) to retain a longitudinal position of the dose limiter (130) relative to the second component (120) when the first component (110) is subject to a longitudinal movement in a proximal direction relative to the second component (120).

2. The dose limiting mechanism according to claim 1, wherein the first profile section (131) comprises a first counter stop face (133) facing in the proximal direction (103) and configured to engage with the stop faces (114, 114', 114") of the first profiled structure (122).

3. The dose limiting mechanism according to any one of the preceding claims, wherein the second profiled structure (122) comprises a number of retaining faces (124, 124', 124") consecutively arranged in longitudinal direction and facing in the distal direction.

4. The dose limiting mechanism according to claim 3, wherein the second profile section (132) comprises a second counter stop face (134) facing in the proximal direction and configured to engage with the retaining faces (124, 124', 124") of the second profiled structure (132).

5. The dose limiting mechanism according to any one of the preceding claims, wherein the first elongated component (110) and the second elongated component (120) being rotatable relative to each other during setting of the dose and wherein the first elongated component (120) and the second elongated component (120) being rotationally locked during dispensing of the dose.

6. The dose limiting mechanism according to any one of the preceding claims, wherein the dose limiter (130) is rotationally locked to one of the first elongated component (110) and the second elongated component (120) and wherein the dose limiter (130) is rotatable to the other one of the first elongated component (110) and the second elongated component (120).

7. The dose limiting mechanism according to any one of the preceding claims, wherein at least one of the first elongated component (110) and the second elongated component (120) comprises an end stop (115, 125) configured to engage with the dose limiter (130).

8. The dose limiting mechanism according to claim 7, wherein the end stop (115) of the first elongated component (110) is configured to prevent a proximally directed longitudinal movement of the first elongated component (110) relative to the dose limiter (130) or relative to the second elongated component (120) when engaged with the dose limiter (130).

9. The dose limiting mechanism according to claim 7 or 8, wherein the end stop (125) of the second elongated component (120) is configured to prevent a distally directed longitudinal movement of the first elongated component (110) relative to the dose limiter (130) or relative to the second elongated component (120) when engaged with the dose limiter (130) .

10. The dose limiting mechanism according to any one of the preceding claims 7 to 9, wherein the end stop (115, 125) and the dose limiter (130) are configured to rotationally interlock when the dose limiter (130) overlaps in longitudinal direction with the end stop (115, 125).

11. The dose limiting mechanism according to claim 10, wherein one of the end stop (115) and the dose limiter (130) comprises at least one locking tooth (116) and wherein the other one of the end stop (115) and the dose limiter (130) comprises a gear ring (146) to rotationally engage with the at least one locking tooth (116).

12. The dose limiting mechanism according to any one of the preceding claims, wherein the first elongated component (110) comprises a drive sleeve (21) movable from a first longitudinal position to a second longitudinal position along the distal direction relative to the housing (3) of the injection device (1), wherein when in the first longitudinal position the drive sleeve (21) being rotationally constrained to the housing (3) and wherein when in the second longitudinal position the drive sleeve (21) being free to rotate relative to the housing (3).

13. The dose limiting mechanism according to any one of the preceding claims, wherein the second elongated component (120) comprises a number sleeve (4) being free to rotate relative to the housing (3).

14. An injection device (1) for setting and dispensing of a dose of a medicament, the injection device (1) comprising:
- a housing (3) to accommodate a cartridge (26) filled with the liquid medicament,
- a lead screw (23) to operably engage with the cartridge (26) for expelling of a dose of the medicament from the cartridge (26),
- a dose dial (2) to set a dose of variable size,
- a trigger (18) to trigger dispensing of the dose, and
- a dose limiting mechanism (100) according to any one of the preceding claims, wherein the first elongated component (110) of the dose limiting mechanism (100) is longitudinally engaged with the trigger (18) and wherein the second elongated component (120) of the dose limiting mechanism (100) is longitudinally constrained to the housing (3).

15. The injection device according to claim 14, further comprising the cartridge (26) filled with the liquid medicament and arranged inside the housing (3).

## Patentansprüche

1. Dosisbegrenzungsmechanismus (100) für eine Injektionsvorrichtung (1), wobei der Dosisbegrenzungsmechanismus (100) zur Anordnung innerhalb eines Gehäuses (3) der Injektionsvorrichtung (1) ausgestaltet ist und betreibbar ist, um eine Gesamtzahl von durch die Injektionsvorrichtung abgebbaren Dosen zu begrenzen, wobei der Dosisbegrenzungsmechanismus (100) umfasst:
- eine erste längliche Komponente (110), die sich in einer Längsrichtung erstreckt und eine erste Profilstruktur (112) umfasst, wobei die erste Profilstruktur (122) mehrere Anschlagsflächen (114, 114', 114") umfasst, die in Längsrichtung hintereinander angeordnet sind und in die distale Richtung zeigen,
- eine zweite längliche Komponente (120), die koaxial oder parallel zu der ersten länglichen Komponente (110) ist und eine zweite Profilstruktur (122) umfasst, die zu der ersten Profilstruktur (112) zeigt,
- einen Dosisbegrenzer (130), der zwischen der ersten länglichen Komponente (110) und der zweiten länglichen Komponente (120) angeordnet ist, wobei der Dosisbegrenzer (130) einen ersten Profilabschnitt (131) und einen zweiten Profilabschnitt (132) umfasst,
- wobei der erste Profilabschnitt (131) mit der ersten Profilstruktur (112) in Eingriff bringbar ist, um eine Längsbewegung der ersten länglichen Komponente (110) zu dem Dosisbegrenzer (130) zu übertragen, wenn die erste längliche Komponente (110) während der Abgabe einer Dosis eine Längsbewegung in einer distalen Richtung relativ zu der zweiten länglichen Komponente (120) erfährt, und
- wobei der zweite Profilabschnitt (132) mit der zweiten Profilstruktur (122) in Eingriff bringbar ist, um eine Längsposition des Dosisbegrenzers (130) relativ zu der zweiten Komponente (120) beizubehalten, wenn die erste Komponente (110) eine Längsbewegung in einer proximalen Richtung relativ zu der zweiten Komponente (120) erfährt.

2. Dosisbegrenzungsmechanismus nach Anspruch 1, wobei der erste Profilabschnitt (131) eine erste Gegenanschlagsfläche (133) umfasst, die in die proximale Richtung (103) zeigt und zum Eingriff mit den Anschlagsflächen (114, 114', 114") der ersten Profilstruktur (122) ausgestaltet ist.

3. Dosisbegrenzungsmechanismus nach einem der vorstehenden Ansprüche, wobei die zweite Profilstruktur (122) mehrere Halteflächen (124, 124', 124") umfasst, die in Längsrichtung hintereinander angeordnet sind und in die distale Richtung zeigen.

4. Dosisbegrenzungsmechanismus nach Anspruch 3, wobei der zweite Profilabschnitt (132) eine zweite Gegenanschlagsfläche (134) umfasst, die in die proximale Richtung zeigt und zum Eingriff mit den Halteflächen (124, 124', 124'') der zweiten Profilstruktur (132) ausgestaltet ist.

5. Dosisbegrenzungsmechanismus nach einem der vorstehenden Ansprüche, wobei die erste längliche Komponente (110) und die zweite längliche Komponente (120) während des Einstellens der Dosis relativ zueinander drehbar sind und wobei die erste längliche Komponente (120) und die zweite längliche Komponente (120) während des Ausgebens der Dosis drehverriegelt sind.

6. Dosisbegrenzungsmechanismus nach einem der vorstehenden Ansprüche, wobei der Dosisbegrenzer (130) mit einer von der ersten länglichen Komponente (110) und der zweiten länglichen Komponente (120) drehverriegelt ist und wobei der Dosisbegrenzer (130) gegenüber der anderen von der ersten länglichen Komponente (110) und der zweiten länglichen Komponente (120) drehbar ist.

7. Dosisbegrenzungsmechanismus nach einem der vorstehenden Ansprüche, wobei wenigstens eine von der ersten länglichen Komponente (110) und der zweiten länglichen Komponente (120) einen Endanschlag (115, 125) umfasst, der zum Eingriff mit dem Dosisbegrenzer (130) ausgestaltet ist.

8. Dosisbegrenzungsmechanismus nach Anspruch 7, wobei der Endanschlag (115) der ersten länglichen Komponente (110) dazu ausgestaltet ist, eine proximal gerichtete Längsbewegung der ersten länglichen Komponente (110) relativ zu dem Dosisbegrenzer (130) oder relativ zu der zweiten länglichen Komponente (120) zu verhindern, wenn er mit dem Dosisbegrenzer (130) in Eingriff steht.

9. Dosisbegrenzungsmechanismus nach Anspruch 7 oder 8, wobei der Endanschlag (125) der zweiten länglichen Komponente (120) dazu ausgestaltet ist, eine distal gerichtete Längsbewegung der ersten länglichen Komponente (110) relativ zu dem Dosisbegrenzer (130) oder relativ zu der zweiten länglichen Komponente (120) zu verhindern, wenn er mit dem Dosisbegrenzer (130) in Eingriff steht.

10. Dosisbegrenzungsmechanismus nach einem der vorstehenden Ansprüche 7 bis 9, wobei der Endanschlag (115, 125) und der Dosisbegrenzer (130) zum Drehverriegeln ausgestaltet sind, wenn der Dosisbegrenzer (130) in Längsrichtung mit dem Endanschlag (115, 125) überlappt.

11. Dosisbegrenzungsmechanismus nach Anspruch 10, wobei einer von dem Endanschlag (115) und dem Dosisbegrenzer (130) wenigstens einen Verriegelungszahn (116) umfasst und wobei der andere
von dem Endanschlag (115) und dem Dosisbegrenzer (130) einen Zahnkranz (146) zum Dreheingriff mit dem wenigstens einen Sperrzahn (116) umfasst.

12. Dosisbegrenzungsmechanismus nach einem der vorstehenden Ansprüche, wobei die erste längliche Komponente (110) eine Antriebshülse (21) umfasst, die von einer ersten Längsposition in eine zweite Längsposition entlang der distalen Richtung relativ zu dem Gehäuse (3) der Injektionsvorrichtung (1) bewegbar ist, wobei, wenn in der ersten Längsposition, die Antriebshülse (21) in Drehrichtung an das Gehäuse (3) gehalten ist und wobei, wenn in der zweiten Längsposition, die Antriebshülse (21) relativ zu dem Gehäuse (3) frei drehbar ist.

13. Dosisbegrenzungsmechanismus nach einem der vorstehenden Ansprüche, wobei die zweite längliche Komponente (120) eine Zahlenhülse (4) umfasst, die sich relativ zu dem Gehäuse (3) frei drehen kann.

14. Injektionsvorrichtung (1) zum Einstellen und Ausgeben einer Dosis eines Medikaments, wobei die Injektionsvorrichtung (1) umfasst:
- ein Gehäuse (3) zum Aufnehmen einer Kartusche (26), die mit dem flüssigen Medikament gefüllt ist,
- eine Leitspindel (23) zum funktionsfähigen Eingriff mit der Kartusche (26) zum Ausstoßen einer Dosis des Medikaments aus der Kartusche (26),
- ein Dosisrad (2) zum Einstellen einer Dosis mit variabler Größe,
- einen Auslöser (18) zum Auslösen des Ausgebens der Dosis, und
- einen Dosisbegrenzungsmechanismus (100) nach einem der vorstehenden Ansprüche, wobei die erste längliche Komponente (110) des Dosisbegrenzungsmechanismus (100) in Längsrichtung mit dem Auslöser (18) in Eingriff steht und wobei die zweite längliche Komponente (120) des Dosisbegrenzungsmechanismus (100) in Längsrichtung an das Gehäuse (3) gebunden ist.

15. Injektionsvorrichtung nach Anspruch 14, ferner umfassend die Kartusche (26), die mit dem flüssigen Medikament gefüllt ist und im Inneren des Gehäuses (3) angeordnet ist.

## Revendications

1. Mécanisme de limitation de dose (100) pour un dispositif d'injection (1), le mécanisme de limitation de dose (100) étant configuré pour être agencé à l'intérieur d'un boîtier (3) du dispositif d'injection (1) et pouvant être actionné pour limiter un nombre total de doses pouvant être distribuées par le dispositif d'injection, le mécanisme de limitation de dose (100) comprenant :
- un premier composant allongé (110) s'étendant dans une direction longitudinale et comprenant une première structure profilée (112), la première structure profilée (122) comprenant un certain nombre de faces de butée (114, 114', 114") agencées consécutivement dans la direction longitudinale et orientées dans la direction distale,
- un second composant allongé (120) coaxial ou parallèle au premier composant allongé (110) et comprenant une seconde structure profilée (122) orientée vers la première structure profilée (112),
- un limiteur de dose (130) agencé entre le premier composant allongé (110) et le second composant allongé (120), le limiteur de dose (130) comprenant une première section profilée (131) et une seconde section profilée (132),
- la première section profilée (131) pouvant venir en prise avec la première structure profilée (112) pour transférer un mouvement longitudinal du premier composant allongé (110) au limiteur de dose (130) lorsque le premier composant allongé (110) est soumis à un mouvement longitudinal dans une direction distale par rapport au second composant allongé (120) pendant la distribution d'une dose, et
- la seconde section profilée (132) pouvant venir en prise avec la seconde structure profilée (122) pour maintenir une position longitudinale du limiteur de dose (130) par rapport au second composant (120) lorsque le premier composant (110) est soumis à un mouvement longitudinal dans une direction proximale par rapport au second composant (120).

2. Mécanisme de limitation de dose selon la revendication 1, la première section profilée (131) comprenant une première face de contre-butée (133) orientée dans la direction proximale (103) et configurée pour venir en prise avec les faces de butée (114, 114', 114") de la première structure profilée (122).

3. Mécanisme de limitation de dose selon l'une quelconque des revendications précédentes, la seconde structure profilée (122) comprenant un certain nombre de faces de retenue (124, 124', 124") agencées consécutivement dans la direction longitudinale et orientées dans la direction distale.

4. Mécanisme de limitation de dose selon la revendication 3, la seconde section profilée (132) comprenant une seconde face de contre-butée (134) orientée dans la direction proximale et configurée pour venir en prise avec les faces de retenue (124, 124', 124") de la seconde structure profilée (132).

5. Mécanisme de limitation de dose selon l'une quelconque des revendications précédentes, le premier composant allongé (110) et le second composant allongé (120) pouvant tourner l'un par rapport à l'autre pendant le réglage de la dose et le premier composant allongé (120) et le second composant allongé (120) étant bloqués en rotation pendant la distribution de la dose.

6. Mécanisme de limitation de dose selon l'une quelconque des revendications précédentes, le limiteur de dose (130) étant verrouillé en rotation sur l'un du premier composant allongé (110) et du second composant allongé (120) et le limiteur de dose (130) pouvant tourner par rapport à l'autre du premier composant allongé (110) et du second composant allongé (120).

7. Mécanisme de limitation de dose selon l'une quelconque des revendications précédentes, au moins l'un du premier composant allongé (110) et du second composant allongé (120) comprenant une butée d'extrémité (115, 125) configurée pour venir en prise avec le limiteur de dose (130).

8. Mécanisme de limitation de dose selon la revendication 7, la butée d'extrémité (115) du premier composant allongé (110) étant configurée pour empêcher un mouvement longitudinal dirigé vers l'intérieur du premier composant allongé (110) par rapport au limiteur de dose (130) ou par rapport au second composant allongé (120) lorsqu'il est en prise avec le limiteur de dose (130).

9. Mécanisme de limitation de dose selon la revendication 7 ou 8, la butée d'extrémité (125) du second composant allongé (120) étant configurée pour empêcher un mouvement longitudinal dirigé distalement du premier composant allongé (110) par rapport au limiteur de dose (130) ou par rapport au second composant allongé (120) lorsqu'il est en prise avec le limiteur de dose (130).

10. Mécanisme de limitation de dose selon l'une quelconque des revendications 7 à 9 précédentes, la butée d'extrémité (115, 125) et le limiteur de dose (130) étant configurés pour s'enclencher par rotation lorsque le limiteur de dose (130) chevauche dans la direction longitudinale la butée d'extrémité (115, 125).

11. Mécanisme de limitation de dose selon la revendication 10, l'un de la butée d'extrémité (115) et du limiteur de dose (130) comprenant au moins une dent de verrouillage (116) et l'autre
de la butée d'extrémité (115) et du limiteur de dose (130) comprenant une couronne dentée (146) destinée à venir en prise en rotation avec l'au moins une dent de verrouillage (116).

12. Mécanisme de limitation de dose selon l'une quelconque des revendications précédentes, le premier composant allongé (110) comprenant un manchon d'entraînement (21) mobile d'une première position longitudinale à une seconde position longitudinale le long de la direction distale par rapport au boîtier (3) du dispositif d'injection (1), lorsqu'il se trouve dans la première position longitudinale, le manchon d'entraînement (21) étant contraint en rotation par rapport au boîtier (3) et, lorsqu'il se trouve dans la seconde position longitudinale, le manchon d'entraînement (21) étant libre de tourner par rapport au boîtier (3).

13. Mécanisme de limitation de dose selon l'une quelconque des revendications précédentes, le second composant allongé (120) comprenant un manchon de numérotation (4) libre de tourner par rapport au boîtier (3).

14. Dispositif d'injection (1) pour régler et distribuer une dose d'un médicament, le dispositif d'injection (1) comprenant :
- un boîtier (3) destiné à recevoir une cartouche (26) remplie du médicament liquide,
- une vis mère (23) destinée à venir en prise de manière opérationnelle avec la cartouche (26) pour expulser une dose du médicament de la cartouche (26),
- un cadran de dosage (2) pour régler une dose de taille variable,
- une gâchette (18) pour déclencher la distribution de la dose, et
- un mécanisme de limitation de dose (100) selon l'une quelconque des revendications précédentes, le premier composant allongé (110) du mécanisme de limitation de dose (100) étant en prise longitudinalement avec la gâchette (18) et le second composant allongé (120) du mécanisme de limitation de dose (100) étant contraint longitudinalement par rapport au boîtier (3).

15. Dispositif d'injection selon la revendication 14, comprenant en outre la cartouche (26) remplie du médicament liquide et agencée à l'intérieur du boîtier (3).
